# EUROPEAN PATENT SPECIFICATION

(11) **EP 1 325 920 B1**
(45) Date of publication and mention of the grant of the patent: **25.08.2010**
(21) Application number: 01967708.7
(22) Date of filing: 17.09.2001
(51) Int. Cl.: C07D 333/62, C07D 409/12, C07D 413/12, C07D 417/12, A61K 31/381, A61K 31/4025, A61K 31/4178, A61K 31/422, A61K 31/427, A61P 43/00, A61P 9/10

(54) **N-SUBSTITUTED BENZOTHIOPHENESULFONAMIDE DERIVATIVES**
N-SUBSTITUIERTE BENZOTHIOPHENSULFONAMID-DERIVATE
DERIVES DE BENZOTHIOPHENESULFONAMIDE SUBSTITUE EN N

(30) Priority: 18.09.2000 JP 2000282046; 20.04.2001 JP 2001122972
(43) Date of publication of application: 09.07.2003
(73) Proprietor: TOA Eiyo Ltd., Tokyo 104-0032 (JP)
(72) Inventor: SATOH, Shoji, TOA Eiyo Ltd., Tokyo Res. Lab., Saitama-shi, Saitama 330-0834 (JP); TATSUI, Akira, TOA Eiyo Ltd. Fukushima Res. Lab, Fukushima-shi, Fukushima 960-0211 (JP); HASEGAWA, Takeshi, TOA Eiyo Ltd. Fukushima Res. Lab., Fukushima-shi, Fukushima 960-0211 (JP); YAMADA, Hideki, TOA Eiyo Ltd. Tokyo Res. Lab., Saitama-shi, Saitama 330-0834 (JP); KAZAYAMA, Shin-ichi, TOA Eiyo Ltd. Tokyo Res. Lab., Saitama-shi, Saitama 330-0834 (JP); MORITA, Takahiro, TOA Eiyo Ltd. Tokyo Res. Lab., Saitama-shi, Saitama 330-0834 (JP); MASAKI, Hidekazu, TOA Eiyo Ltd. Tokyo Res.Lab., Saitama-shi, Saitama 330-0834 (JP); TAKAHASHI, Atsuo, TOA Eiyo Ltd. Tokyo Res. Lab., Saitama-shi, Saitama 330-0834 (JP)
(74) Representative: Albrecht, Thomas
(86) International application number: PCT/JP2001/008061
(87) International publication number: WO 2002/022595

(56) References cited:
- WO-A-00/10982
- WO-A-00/12073
- WO-A-98/27081
- WO-A-99/37623
- WO-A1-96/31492
- WO-A1-97/11941
- WO-A2-00/12623
- WO-A2-99/42465
- JP-A- 2000 095 770
- JP-A- 2001 097 946
- DATABASE CA [Online] CHEMICAL ABSTRACTS SERVICE, COLUMBUS, OHIO, US; 1962, PAILER, M. ET AL: "Sulfonation of thionaphthene and methylthionaphthenes" XP002308847 retrieved from STN Database accession no. 1962:2297 & MONATSHEFTE FUER CHEMIE ( 1961 ), 92, 677-83 CODEN: MOCMB7; ISSN: 0026-9247, 1961,

## Description

### FIELD OF THE INVENTION

The present invention relates to medicaments, especially N-substituted benzothiophenesulfonamide derivatives or salts thereof which selectively inhibit chymase, and chymase inhibitors containing the same as the active ingredient. Since the compounds have a selective inhibitory action on chymase, they are useful as agents for preventing or treating hypertension, hypercardia, cardiac failure, cardiac infarction, arteriosclerosis, diabetic or non-diabetic renal diseases, diabetic retinopathy, restenosis after percutaneous transluminal coronary angioplasty (hereinafter, abbreviated as PTCA), intimal thickening after bypass grafting, ischemic re-perfusion disorder, chronic rheumatism, keloid, psoriasis, allergy, inflammation, asthma, atopic dermatitis, solid tumors caused by abnormal increase of production of angiotensin II (hereinafter, abbreviated as Ang II) or endothelin I (hereinafter, abbreviated as ET-1) based on chymase activity.

### BACKGROUND OF THE INVENTION

Since Ang II and ET-1 have a cell growth- accelerating action in addition to a blood pressure- elevating action, they are considered as causative agents or risk factors for diseases such as hypertension, hypercardia, cardiac infarction, arteriosclerosis, diabetic or non-diabetic renal diseases and restenosis after PTCA. Moreover, it is known that Ang II is formed from angiotensin I (hereinafter, abbreviated as Ang I) by angiotensin converting enzyme (hereinafter, abbreviated as ACE), and a large number of ACE inhibitors have been developed as agents for preventing or treating the above diseases. On the other hand, it is known that ET-1 is a physiologically active peptide composed of 21 amino acid residues (hereinafter, abbreviated as ET(1-21)) which is formed from big endothelin (hereinafter, abbreviated as Big ET-1) by endothelin converting enzyme (hereinafter, abbreviated as ECE), but ECE inhibitors and ET-1 receptor antagonists are still in developmental stages as medicaments.

Recently, in addition to ACE, an enzyme producing Ang II from Ang I has been discovered and named chymase. Urata et al. purified chymase from human heart and has shown that 70 to 80% amount of Ang II produced in heart and blood vessels was due to chymase (J. Biol. Chem., 265, 22348 (1990). Moreover, when the fact that no effectiveness of ACE inhibitors on restenosis after PTCA is observed [MERCAPTOR study (Circulation, 86(1), 100 (1992)) and MARCAPTOR study (J. Am. Coll. Cardiol., 27(1), p. 1 (1996))] and the fact that chymase inhibitors are effective on a canine intimal thickening model of grafted blood vessel using jugular vein (Miyazaki, Takai et al.; Febs. Lett., 467, 141 (2000)) are together considered, it is important to inhibit chymase rather than ACE for preventing and treating cardiac and circulatory diseases caused by abnormal increase of the production of Ang II and thus the application of chymase inhibitors to cardiac and circulatory diseases is suggested.

Furthermore, in the recent past, it has been revealed that chymase specifically degrades Big ET-1 into a physiologically active peptide composed of 31 amino acid residues (hereinafter, abbreviated as ET(1-31)). It has been reported that the ET(1-31) acts on the receptor on which original ET(1-21) acts, to cause bronchoconstriction and vasoconstriction (Kido et al.; J. Immunol., 159, 1987 (1997)). In this connection, with regard to the concentration in human blood, both of ET(1-31) and ET(1-21) have about the same distribution and activity, and after cardiac infarction, ET(1-31) increases more largely than ET(1-21) does, which is maintained for two weeks after the incidence (Tamaki, Nishisu et al.; Jpn. J. Pharmacol., 82(suppl I), 26 (2000)), and the fact suggests importance of inhibition of chymase and application of chymase inhibitors to cardiac and circulatory diseases.

Accordingly, chymase is considered to participate in production and degradation of physiologically active peptides, remodeling of extracellular matrix, network with cytokine, immunity, and the like and contribute to restoration of metabolic turnover. Thus, a chymase inhibitor is expected to apply to cardiac and circulatory diseases.

Moreover, as a result of administration of Ang II into a sponge in a hamster subdermally sponge-implanted model, removal of the sponge after 7 days, and measurement of hemoglobin content, vascularization was observed (mainly capillary vessels). When ovalbumin (10 µg/site/day) as an antigen is administered to a sensitized animal via sponge, vascularization occurs as in the case of Compound 48/80.

This vascularization was also inhibited by chymostatin (Muramatsu et al.; J. Biol. Chem., 275(8), 5545 (2000)). The above results indicate that activation of mast cells by antigen stimulation can also cause vascularization, and chymase may be involved in this process. Thus, new roles of chymase are suggested in a variety of inflammatory allergy diseases. From such a viewpoint, a chymase inhibitor is expected to exhibit effects on solid tumors, diabetic retinopathy, rheumatoid arthritis and atherosclerosis.

Currently, as inhibitors against chymase, peptide-type chymase inhibitors are disclosed in JP-A-10-7661, JP-A-11-49739, JP-A-11-246437, WO98/09949, WO98/18794, WO99/32459 and WO00/06594. On the other hand, non-peptide-type chymase inhibitors are disclosed in JP-A-10-87493, JP-A-10-245384, JP-A-12-95770, WO96/04248, WO97/11941, WO99/09977 WO00/03997, WO00/10982, WO00/32587. However, until now, no clinically applicable chymase inhibitor has been found. Accordingly, it is desired to develop a clinically applicable chymase inhibitor which enables prevention and treatment of cardiac and circulatory diseases caused by abnormal increase of production of Ang II and ET-1.

### DISCLOSURE OF THE INVENTION

As a result of the extensive studies for achieving the above objects, the present inventors have found that an N-substituted benzothiophenesulfonamide derivative or a pharmaceutically acceptable salt thereof has an excellent human chymase inhibitory activity and enzyme selectivity, and is stable even in rat blood plasma.

Namely, the invention relates to an N-substituted benzothiophenesulfonamide derivative represented by formula (I): 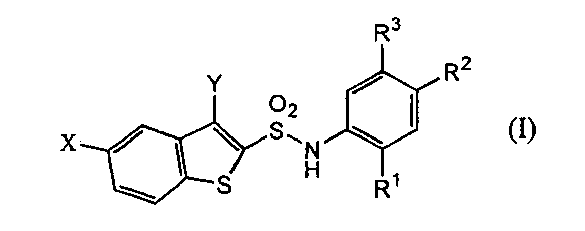 wherein X represents a hydrogen atom, a halogen atom or a lower alkyl group having 1 to 4 carbon atoms;
Y represents a lower alkyl group having 1 to 4 carbon atoms; R¹ and R² each may be the same or different and represents a hydrogen atom, a lower alkoxycarbonyl group having 1 to 4 carbon atoms in the alkoxy residue, a lower alkylsulfonyl group having 1 to 4 carbon atoms, a benzoyl group, an acyl group having 1 to 4 carbon atoms, a lower alkoxy group having 1 to 4 carbon atoms, a lower alkoxycarbonylmethylthioacetyl group having 1 to 4 carbon atoms in the alkoxy residue, a nitro group, -CONHR⁴ in which R⁴ represents a hydrogen atom, a lower alkoxycarbonylmethyl group having 1 to 4 carbon atoms in the alkoxy residue, a carboxymethyl group or -CH(CH₂OH)COOR⁵ in which R⁵ represents a hydrogen atom or a lower alkyl group having 1 to 4 carbon atoms, a group represented by formula: 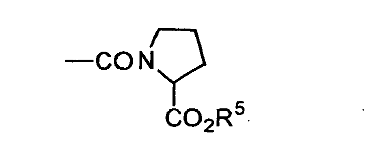 in which R⁵ has the same meaning as above, a monocyclic heterocyclic group represented by formulae which may be substituted by -CO₂R⁵ in which R⁵ has the same meaning as above: 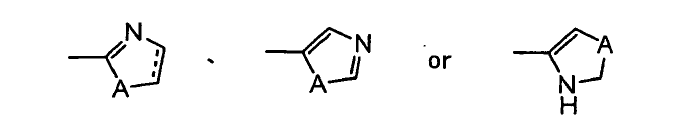 in which A represents an oxygen atom, a sulfur atom or NH and the dotted part represents a single bond or a double bond, a hydroxy lower alkyl group having 1 to 4 carbon atoms, a cyano group provided that R¹ and R² are not hydrogen atoms at the same time; and R³ represents a hydrogen atom, a lower alkoxy group having 1 to 4 carbon atoms or a lower alkyl group having 1 to 4 carbon atoms, or a salt thereof.

The N-substituted benzothiophenesulfonamide derivative represented by formula (I) or a pharmaceutically acceptable salt thereof according to the invention has a strong inhibitory activity against chymase and is a extremely useful compound for preventing or treating cardiac or circulatory diseases caused by abnormal increase of production of Ang II or ET-1 based on chymase activity.

Also, it has been found that compounds represented by formulae 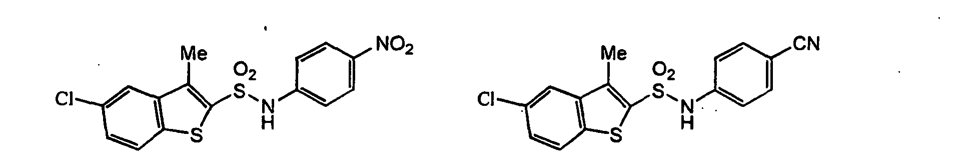 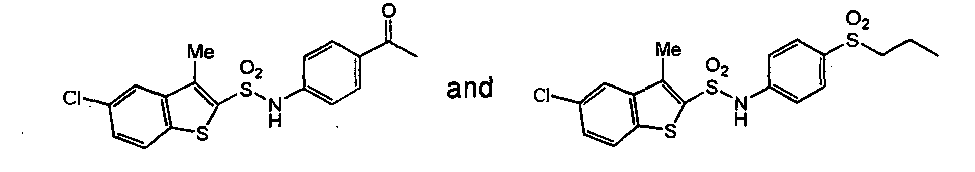 have a strong inhibitory activity against chymase and are extremely useful for preventing or treating cardiac or circulatory diseases caused by abnormal increase of production of Ang II or ET-1 based on chymase activity.

### BEST MODE FOR CARRYING OUT THE INVENTION

Examples of the halogen atom for X include a fluorine atom, a chlorine atom, a bromine atom or an iodine atom, and particularly, a fluorine atom or a chlorine atom is preferable.

Examples of the lower alkyl group having 1 to 4 carbon atoms for X include a methyl group, an ethyl group, a propyl group, an isopropyl group, a butyl group, an isobutyl group, a sec-butyl group or a tert-butyl group, and particularly, a methyl group or an ethyl group is preferable.

Examples of the lower alkyl group having 1 to 4 carbon atoms for Y include a methyl group, an ethyl group, a propyl group, an isopropyl group, a butyl group, an isobutyl group, a sec-butyl group or a tert-butyl group, and particularly, a methyl group or an ethyl group is preferable.

Examples of the lower alkoxycarbonyl group having 1 to 4 carbon atoms in the alkoxy residue for R¹ and R² include a methoxycarbonyl group, an ethoxycarbonyl group, a propoxycarbonyl group, an isopropoxycarbonyl group, a butoxycarbonyl group, an isobutoxycarbonyl group, a sec-butoxycarbonyl group or a tert-butoxycarbonyl group, and particularly, a methoxycarbonyl group, an ethoxycarbonyl group, an isopropoxycarbonyl group or a tert-butoxycarbonyl group is preferable.

Examples of the lower alkylsulfonyl group having 1 to 4 carbon atoms for R¹ and R² include a methanesulfonyl group, an ethanesulfonyl group, a propanesulfonyl group, an isopropanesulfonyl group, a butanesulfonyl group, an isobutanesulfonyl group, a sec-butanesulfonyl group or a tert-butanesulfonyl group, and particularly, a methanesulfonyl group or an ethanesulfonyl group is preferable.

Examples of the acyl group having 1 to 4 carbon atoms for R¹ and R² include a formyl group, an acetyl group, a propionyl group, a butyryl group or an isobutyryl group, and particularly, an acetyl group is preferable.

Examples of the lower alkoxy group having 1 to 4 carbon atoms for R¹, R² and R³ include a methoxy group, an ethoxy group, a propoxy group, an isopropoxy group, a butoxy group, an isobutoxy group, a sec-butoxy group or a tert-butoxy group, and particularly, a methoxy group or an ethoxy group is preferable.

Examples of the lower alkoxycarbonylmethylthioacetyl group having 1 to 4 carbon atoms in the alkoxy residue for R¹ and R² include a methoxycarbonylmethylthioacetyl group, an ethoxycarbonylmethylthioacetyl group, a propoxycarbonylmethylthioacetyl group, an isopropoxycarbonylmethylthioacetyl group, a butoxycarbonylmethylthioacetyl group, an isobutoxycarbonylmethylthioacetyl group, a sec-butoxycarbonylmethylthioacetyl group or a tert-butoxycarbonylmethylthioacetyl group, and particularly, a methoxycarbonylmethylthioacetyl group or an ethoxycarbonylmethylthioacetyl group is preferable.

In the case that R¹ and R² each is -CONHR⁴, examples of the lower alkoxycarbonylmethyl group having 1 to 4 carbon atoms in the alkoxy residue for R⁴ include a methoxycarbonylmethyl group, an ethoxycarbonylmethyl group, a propoxycarbonylmethyl group, an isopropoxycarbonylmethyl group, a butoxycarbonylmethyl group, an isobutoxycarbonylmethyl group, a sec-butoxycarbonylmethyl group or a tert-butoxycarbonylmethyl group, and particularly, a methoxycarbonylmethyl group, an ethoxycarbonylmethyl group or an isopropoxycarbonylmethyl group is preferable.

In the case that R¹ and R² each is -CONHR⁴ and R⁴ is -CH(CH₂OH)COOR⁵, examples of the lower alkyl group having 1 to 4 carbon atoms for R⁵ include a methyl group, an ethyl group, a propyl group, an isopropyl group, a butyl group, an isobutyl group, a sec-butyl group or a tert-butyl group, and particularly, a methyl group or an ethyl group is preferable.

In the case that R¹ and R² each is a group represented by formula: 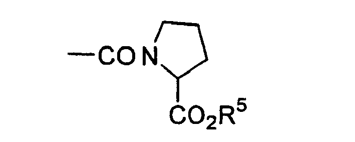 and R⁵ is a lower alkyl group having 1 to 4 carbon atoms, examples of the lower alkyl group having 1 to 4 carbon atoms for R⁵ is the same meaning as above.

In the case that R¹ and R² each is a monocyclic heterocyclic group represented by formulae which may be substituted by -CO₂R⁵: 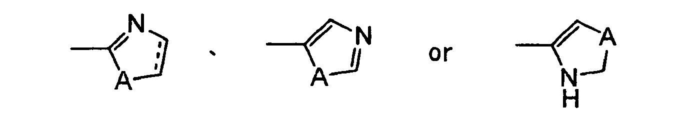 examples of the lower alkyl group for R⁵ is the same meaning as above.

Examples of the monocyclic heterocylic group represented by the formulae which may be substituted: 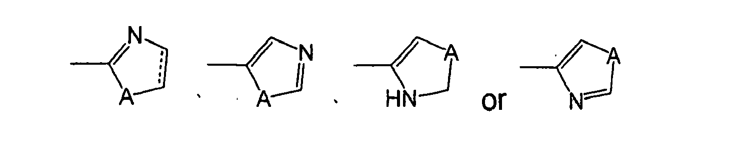 in which A represents an oxygen atom, a sulfur atom or NH and the dotted part represents a single bond or a double bond, include those represented by the following formulae.

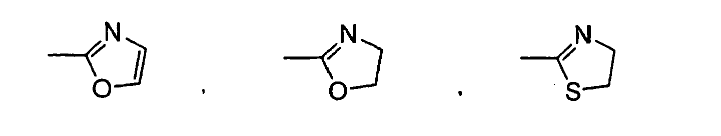

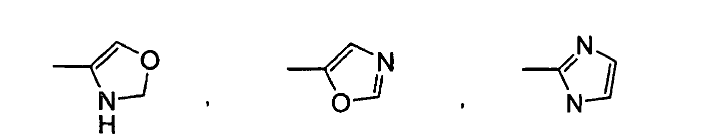

Specific examples preferably include those represented by the formulae: 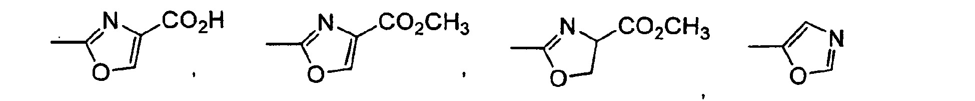 and these substituents are preferably substituted as R². In this case, it is further preferable that R¹ is a methanesulfonyl group and R³ is a hydrogen atom.

Examples of the hydroxy lower alkyl group having 1 to 4 carbon atoms for R¹ and R² include a linear or branched hydroxy lower alkyl group having 1 to 4 carbon atoms such as a hydroxymethyl group, a hydroxyethyl group, a hydroxypropyl group or a hydroxybutyl group, and particularly, a hydroxymethyl group, a 1-hydroxyethyl group or a 2-hydroxyethyl group is preferable.

Examples of the lower alkyl group having 1 to 4 carbon atoms for R³ include a methyl group, an ethyl group, a propyl group, an isopropyl group, a butyl group, an isobutyl group, a sec-butyl group or a tert-butyl group, and particularly, a methyl group or an ethyl group is preferable.

In this regard, examples of specific compounds include methyl 4-(5-chloro-3-methylbenzo[b]thiophene-2-sulfonylamino)-3-methanesulfonylbenzoate, sodium methyl 4-(5-chloro-3-methylbenzo[b]thiophene-2-sulfonylamino)-3-methanesulfonylbenzoate, isopropyl 4-(5-chloro-3-methylbenzo[b]thiophene-2-sulfonylamino)-3-methanesulfonylbenzoate, 5-chloro-3-methylbenzo[b]thiophene-2-sulfonic acid (4-acetyl-2-methanesulfonylphenyl)amide, 5-chloro-3-methylbenzo[b]thiophene-2-sulfonic acid (4-benzoyl-2-methanesulfonylphenyl)amide, ethyl 4-(5-chloro-3-methylbenzo[b]thiophene-2-sulfonylamino)-3-methanesulfonylbenzoate, tert-butyl 4-(5-chloro-3-methylbenzo[b]thiophene-2-sulfonylamino)-3-methanesulfonylbenzoate, methyl 4-(5-chloro-3-methylbenzo[b]thiophene-2-sulfonylamino)-3-ethanesulfonylbenzoate, methyl 4-(5-chloro-3-methylbenzo[b]thiophene-2-sulfonylamino)-5-methanesulfonyl-2-methylbenzoate, dimethyl 4-(5-chloro-3-methylbenzo[b]thiophene-2-sulfonylamino)isophthalate, methyl 4-(5-chloro-3-methylbenzo[b]thiophene-2-sulfonylamino)-3-methoxybenzoate, methyl 4-(5-chloro-3-methylbenzo[b]thiophene-2-sulfonylamino)-3-nitrobenzoate, ethyl 4-(5-chloro-3-methylbenzo[b]thiophene-2-sulfonylamino) benzoate, 5-chloro-3-methylbenzo[b]thiophene-2-sulfonic acid (2,4-dimethanesulfonylphenyl)amide, 5-chloro-3-methylbenzo[b]thiophene-2-sulfonic acid (4-acetyl-2-nitrophenyl)amide, 5-chloro-3-methylbenzo[b]thiophene-2-sulfonic acid (4-hydroxymethyl-2-methanesulfonylphenyl)amide, 5-chloro-3-methylbenzo[b]thiophene-2-sulfonic acid (4-benzoylphenyl)amide, 5-chloro-3-methylbenzo[b]thiophene-2-sulfonic acid (2-methanesulfonylphenyl)amide, methyl 4-(5-fluoro-3-methylbenzo[b]thiophene-2-sulfonylamino)-3-methanesulfonylbenzoate, methyl 4-(5-methyl-3-methylbenzo[b]thiophene-2-sulfonylamino)-3-methanesulfonylbenzoate, 5-fluoro-3-methylbenzo[b]thiophene-2-sulfonic acid (4-acetyl-2-methanesulfonylphenyl) amide, methyl 4-(3-methylbenzo[b]thiophene-2-sulfonylamino)-3-methanesulfonylbenzoate, methyl 2-[4-(5-chloro-3-methylbenzo[b]thiophene-2-sulfonylamino)-3-methanesulfonylphenyl]oxazole-4-carboxylate, methyl 2-[4-(5-fluoro-3-methylbenzo[b]thiophene-2-sulfonylamino)-3-methanesulfonylphenyl]oxazole-4-carboxylate, 2-[4-(5-chloro-3-methylbenzo[b]thiophene-2-sulfonylamino)-3-methanesulfonylphenyl]oxazole-4-carboxylic acid, 2-[4-(5-fluoro-3-methylbenzo[b]thiophene-2-sulfonylamino)-3-methanesulfonylphenyl]oxazole-4-carboxylic acid, disodium 2-[4-(5-chloro-3-methylbenzo[b]thiophene-2-sulfonylamino)-3-methanesulfonylphenyl]oxazole-4-carboxylate, disodium 2-[4-(5-fluoro-3-methylbenzo[b]thiophene-2-sulfonylamino)-3-methanesulfonylphenyl]oxazole-4-carboxylate.

Of the above-described compounds, methyl 4-(5-chloro-3-methylbenzo[b]thiophene-2-sulfonylamino)-3-methanesulfonylbenzoate, sodium methyl 4-(5-chloro-3-methylbenzo[b]thiophene-2-sulfonylamino)-3-methanesulfonylbenzoate, isopropyl 4- (5-chloro-3-methylbenzo[b]thiophene-2-sulfonylamino)-3-methanesulfonylbenzoate, 5-chloro-3-methylbenzo[b]thiophene-2-sulfonic acid (4-acetyl-2-methanesulfonylphenyl)amide, 5-chloro-3-methylbenzo[b]thiophene-2-sulfonic acid (4-benzoyl-2-methanesulfonylphenyl)amide, methyl 4-(5-fluoro-3-methylbenzo[b]thiophene-2-sulfonylamino)-3-methanesulfonylbenzoate, methyl 4-(5-methyl-3-methylbenzo[b]thiophene-2-sulfonylamino)-3-methanesulfonylbenzoate, 5-fluoro-3-methylbenzo[b]thiophene-2-sulfonic acid (4-acetyl-2-methanesulfonylphenyl)amide, methyl 4-(3-methylbenzo[b]thiophene-2-sulfonylamino)-3-methanesulfonylbenzoate, 2-[4-(5-chloro-3-methylbenzo[b]thiophene-2-sulfonylamino)-3-methanesulfonylphenyl]oxazole-4-carboxylic acid, 2-[4-(5-fluoro-3-methylbenzo[b]thiophene-2-sulfonylamino)-3-methanesulfonylphenyl]oxazole-4-carboxylic acid, disodium 2-[4-(5-chloro-3-methylbenzo[b]thiophene-2-sulfonylamino)-3-methanesulfonylphenyl]oxazole-4-carboxylate, disodium 2-[4-(5-fluoro-3-methylbenzo[b]thiophene-2-sulfonylamino)-3-methanesulfonylphenyl]oxazole-4-carboxylate are preferable.

The following will describe the process for producing the N-substituted benzothiophenesulfonamide derivative or salt thereof of the invention. The compound of the general formula (I) of the invention can be produced through the production process as illustrated by the following reaction scheme.

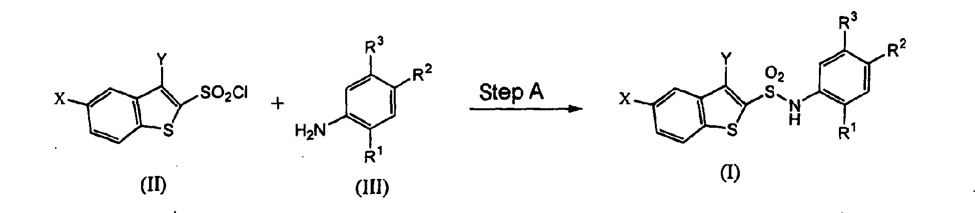

That is, in the scheme, the compound can be produced by reacting an amine represented by the formula (III) (in which R¹, R² and R³ have the same meaning as defined in the formula (I)) with an sulfonyl chloride (II) in the presence of a base such as sodium amide, lithium amide, sodium hydride, potassium carbonate, potassium tert-butoxide, triethylamine, ethyldiisopropylamine, pyridine, or 1,8-diazabicyclo[5.4.0]undec-7-ene (hereinafter, abbreviated as DBU) in a solvent such as dioxane, tetrahydrofuran (hereinafter, abbreviated as THF), acetone, dimethylformamide (hereinafter, abbreviated as DMF), dimethyl sulfoxide (hereinafter, abbreviated as DMSO), chloroform, pyridine or a mixed solvent thereof within the range of -10°C to a boiling point of the solvent.

In this connection, in the case of the compound wherein R¹ and/or R² have an ester group, the compound of formula (I) can be further produced by reducing the ester group to form a hydroxymethyl group.

Moreover, in the case of the compound wherein R¹ and/or R² is -CONHR⁴ and R⁴ is a lower alkoxycarbonylmethyl group having 1 to 4 carbon atoms in the alkoxy residue, the compound of formula (I) can be further produced after subjecting the compound to ester hydrolysis, and salt formation thereof can be also conducted.

Furthermore, for example, in the case of conducting the monocyclic heterocylic group as above, specifically the following group for R², 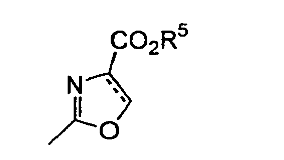 the following steps can be sequentially carried out.

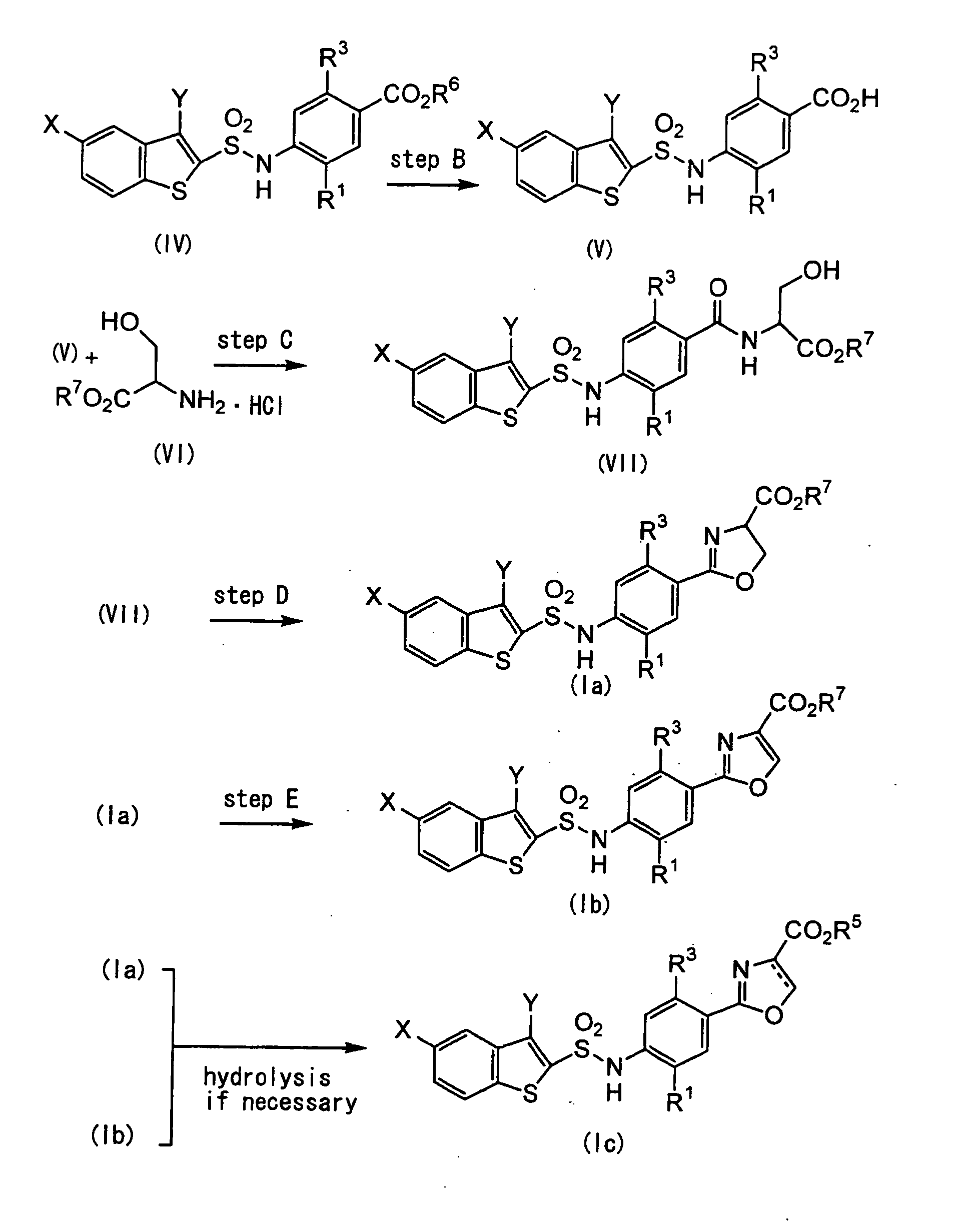

A compound (V) wherein R² is CO₂H can be produced by subjecting a compound (IV) wherein R² is CO₂R⁶ (R⁶ represents a lower alkyl group) to ester hydrolysis (Step B). Thereafter, the compound (VII) is obtained by reacting the compound (V) with serine ester hydrochloride (VI) wherein R⁷ represents a lower alkyl group, in the presence of a base such as triethylamine, ethyldiisopropylamine or DBU using a condensing agent such as N,N'-dicyclohexylcarbodiimide or 1-ethyl-3-(3-dimethylaminopropylcarbodiimide (hereinafter, abbreviated as EDC) (Step C), and then a compound (Ia) and a compound (Ib) are obtained in accordance with the method known in literatures (Tetrahedron Letters, 33, 907 (1992), J. Org. Chem., 38, 26 (1973), J. Org. Chem., 58, 4494 (1993), Org. Lett., 2, 1165 (2000)) (Steps F and G), whereby the production is completed.

Furthermore, the compound (Ia) and the compound (Ib) are subjected to ester hydrolysis, if necessary. Thus, the compound represented by formula (Ic) can be produced. Moreover, salt formation thereof can be conducted.

The thus formed compound of formula (I) can be isolated and purified by conventional methods such as recrystallization and column chromatography.

The present invention includes a salt of the compound of formula (I). Examples of the salt of the compound of formula (I) are preferably pharmaceutically acceptable salts in view of use for a medicament.

Specific examples of the salt include a pharmaceutically acceptable salt with an acid or base, e.g., a salt with an inorganic acid such as hydrochloride, hydrobromide, hydroiodide, sulfate, nitrate or phosphate; a salt with an organic acid such as acetate, trifluoroacetate, oxalate, fumarate, maleate, tartrate, mesylate or tosylate; a salt with an alkali metal such as sodium salt or potassium salt; or a salt with an alkaline earth metal such as calcium salt depending on the compound, by a usual method.

The compound of formula (I) and a pharmaceutically acceptable salt thereof are useful as a agent for a chymase inhibitor.

The compound of formula (I) sometimes includes optical isomers based on an asymmetric carbon atom. These various types of isomers isolated and mixtures of these isomers are also encompassed within the invention. Moreover, the compound of formula (I) of the invention includes hydrates and various solvates. All the crystal forms are also encompassed within the compound of formula (I).

The invention also includes a medicament containing the N-substituted benzothiophenesulfonamide derivative represented by the above formula (I) or a pharmaceutically acceptable salt thereof. The medicament includes an agent for inhibiting chymase activity.

The medicament is effective for diagnosing, preventing and/or treating diseases caused by abnormal increase of production of angiotensin II or endothelin I.

The above-described diseases include circulatory diseases and inflammatory allergosis.

Specifically, the diseases include hypertension, hypercardia, cardiac failure, cardiac infarction, arteriosclerosis, diabetic or non-diabetic renal disease, diabetic retinopathy, ischemic re-perfusion disorder, restenosis after percutaneous transluminal coronary angioplasty, intimal thickening after bypass grafting, chronic rheumatism, keloid, psoriasis, allergy, inflammation, asthma, atopic dermatitis, or solid tumors.

The compound of formula (I) or a pharmaceutically acceptable salt thereof may be administered orally or parenterally (e.g. injection into a vein or intramuscular injection).

Examples of preparations for oral administration include tablets including sugar-coated tablets and film-coated tablets, pills, granules, powders, capsules including soft capsules, syrups, emulsions, suspensions, and the like.

The preparations for oral administration can be manufactured with mixing additives usually employed in the pharmaceutical field in accordance with a known method. Examples of such additives include an excipient such as lactose, mannitol or anhydrous calcium hydrogen phosphate; a binder such as hydroxypropyl cellulose, methyl cellulose or polyvinylpyrrolidone; a disintegrator such as starch and carboxymethyl cellulose; a lubricant such as magnesium stearate or talc; and the like.

Examples of preparations for parenteral administration include injections.

These injections can be manufactured by a common method, for example, by dissolving the compound of formula (I) or a pharmaceutically acceptable salt thereof in Japanese Pharmacopoeia-grade water for injection. As needed, an isotonic agent such as sodium chloride, a buffering agent such as sodium dihydrogen phosphate or sodium monohydrogen phosphate, or the like may be mixed.

The dose of the compound of formula (I) per day for an adult can be appropriately changed depending on the conditions, body weight, age of an individual patient, and a kind of a compound, route of administration the like, and the dose is suitably from about 1 mg to 1000 mg, preferably about 10 mg to 300 mg in the case of oral administration.

In the case of parenteral administration, the dose may be from one tenth to a half of the dose in the case of oral administration. The dose can be appropriately changed depending on the conditions, body weight, age and the like of an individual patient.

### EXAMPLES

The following will describe the invention in more detail with Reference Examples and Examples, but the invention is not limited thereto.

### Example 1

### Methyl 4-(5-chloro-3-methylbenzo[b]thiophene-2-sulfonylamino)-3-methanesulfonylbenzoate

Into a mixed solvent of 20 mL of THF and 3 mL of DMF was dissolved 985 mg of methyl 4-amino-3-methanesulfonylbenzoate, followed by addition of 170 mg of sodium hydride (oily, 60%) at 0°C. After 20 minutes of stirring at the same temperature, 1.28 g of 5-chloro-2-chlorosulfonyl-3-methylbenzo[b]thiophene was added at 0°C, followed by 1 hour of stirring at room temperature. Further, 150 mg of sodium hydride (oily, 60%) was added at room temperature and the mixture was stirred for 2 hours at the same temperature. After confirmation of disappearance of the starting material, the reaction was terminated by adding saturated aqueous ammonium chloride solution at 0°C, followed by extraction with ethyl acetate. The organic layer was washed with saturated brine and then dried over anhydrous sodium sulfate. The solvent was removed by evaporation and the residue was purified by silica gel chromatography (ethyl acetate/hexane = 3/1) to obtain 911 mg of the title compound as colorless powder.
Melting point: 179-181°C
¹H-NMR (CDCl₃) : δ 2.70 (3H, s) 3.06 (3H, s) 3.90 (3H, s) , 7.48 (1 H, dd, J=2.1, 8.6Hz) ,7.74 (1H, d, J=8.6Hz) ,7.89 (1H, d, J=2.1Hz) ,7. 86 (1H, d, J=8.8Hz) , 8.19 (1H, dd, J=2.0, 8.8Hz), 8.50 (1H, d, J=2.0Hz ) , 9.84 (1H, s).
IR νₘₐₓ (KBr) : 3217, 1720, 1608, 1504, 1442, 1392, 1308, 1165, 1119 c m⁻¹.

### Example 2

### Ethyl 4-(5-chloro-3-methylbenzo[b]thiophene-2-sulfonylamino)-3-methanesulfonylbenzoate

In the same manner as in Example 1, 529 mg of the title compound was obtained as colorless powder from 559 mg of ethyl 4-amino-3-methanesulfonylbenzoate.
Melting point: 167-169°C
¹H-NMR (CDCl₃) : δ 1.36 (3H, t, J=7.1Hz), 2.70 (3H, s), 3.06 (3H,s ) , 4.36 (2H, q, J=7.1Hz) , 7.47 (1H, dd, J=2.0,8.8Hz), 7.74 (1H, d, J= 8.8Hz), 7.78 (1H, d, J=2.0Hz), 7.86 (1H, d, J=8.8Hz), 8.19 (1H, dd, J =2.0,8.8Hz), 8.50 (1H, d, J=2.0Hz), 9.83 (1H, brs).
IR νₘₐₓ (KBr) : 3224, 2985, 1716, 1608, 1500, 1358, 1300, 1142 cm⁻¹.

### Example 3

### Tert-butyl 4-(5-chloro-3-methylbenzo[b]thiophene-2-sulfonylamino)-3-methanesulfonylbenzoate

In the same manner as in Example 1, 148 mg of the title compound was obtained as colorless powder from 128 mg of tert-butyl 4-amino-3-methanesulfonylbenzoate.
Melting point: 236-238°C
¹H-NMR (CDCl₃) : δ 1.54 (9H, s), 2.52 (3H, s), 3.28 (3H,s), 7.55-7 .80 (4H,m), 8.00 (1H, s), 8.25-8.30 (1H, m).
IR νₘₐₓ (KBr): 3467, 2974, 2327, 1705, 1662, 1597, 1477, 1396, 1296, 1130, 1099cm⁻¹.

### Example 4

### Methyl 4-(5-chloro-3-methylbenzo[b]thiophene-2-sulfonylamino)-3-ethanesulfonylbenzoate

In the same manner as in Example 1, 80 mg of the title compound was obtained as colorless powder from 76 mg of methyl 4-amino-3-ethanesulfonylbenzoate.
Melting point: 172-173°C
¹H-NMR (CDCl₃) : δ 1.27 (3H,t,J=7.3Hz) , 2.74 (3H, s), 3.24 (2H,q, J=7.3Hz), 3.77 (3H,s), 7.20-7.31 (2H, m), 7.43-7.56 (3H, m), 8. 31 (1H, s).
IR νₘₐₓ (KBr) : 3482, 3217, 2931, 1709, 1597, 1481, 1439, 1284, 1126 c m⁻¹.

### Example 5

### Methyl 4-(5-chloro-3-methylbenzo[b]thiophene-2-sulfonylamino)-5-methanesulfonyl-2-methylbenzoate

Into 10 mL of THF was dissolved 135 mg of methyl 4-amino-5-methanesulfonyl-2-methylbenzoate, followed by addition of 22 mg of sodium hydride (oily, 60%) at room temperature. After 20 minutes of stirring at the same temperature, 130 mg of 5-chloro-2-chlorosulfonyl-3-methylbenzo[b]thiophene was added at 0°C, followed by 1 hour of stirring at room temperature and 5 hours of heating under refluxing. Further, 1 mL of DMF, 22 mg of sodium hydride (oily, 60%) and 50 mg of 5-chloro-2-chlorosulfonyl-3-methylbenzo[b]thiophene were added and the mixture was heated under refluxing for 2.5 hours. After confirmation of disappearance of the starting material, the reaction was terminated by adding saturated aqueous ammonium chloride solution at 0°C, followed by extraction with ethyl acetate. The organic layer was washed with saturated brine and then dried over anhydrous sodium sulfate. The solvent was removed by evaporation and the residue was purified by silica gel chromatography (ethyl acetate/hexane = 3/2) to obtain 102 mg of the title compound as colorless powder.
Melting point: 205-207°C
¹H-NMR (CDCl₃) : δ 2.65 (3H, s) , 2.71 (3H,s), 3.04 (3H,s) , 3.87 (3 H, s), 7.49 (1H, dd, J=2.0,8.6Hz), 7.68 (1H, s), 7.77 (1H, d, J=8.6Hz ), 7.80 (1H, d, J=2.0Hz), 8.42 (1H,s), 9.73 (1H, s).
IR νₘₐₓ (KBr) : 3259, 1728, 1604, 1554, 1504, 1439, 1385, 1354, 1300, 1 257,1157,1092 cm⁻¹.

### Example 6

### Dimethyl 4-(5-chloro-3-methylbenzo[b]thiophene-2-sulfonylamino isophthalate

Into 8 mL of THF was dissolved 115 mg of dimethyl 4-aminoisophthalate, followed by addition of 22 mg of sodium hydride (oily, 60%). After 20 minutes of stirring at room temperature, 130 mg of 5-chloro-2-chlorosulfonyl-3-methylbenzo[b]thiophene was added at the same temperature, followed by 30 minutes of stirring at room temperature. Further, 26 mg of sodium hydride (oily, 60%) was added and the whole was heated under refluxing for 6 hours. After confirmation of disappearance of the starting material, the reaction was terminated by adding saturated aqueous ammonium chloride solution at 0°C, followed by extraction with ethyl acetate. The organic layer was washed with saturated brine and then dried over anhydrous sodium sulfate. The solvent was removed by evaporation and the residue was purified by silica gel chromatography (ethyl acetate/hexane = 1/1) to obtain 62 mg of the title compound as light yellow amorphous. ¹H-NMR (CDCl₃) :δ 2.64 (3H,s) , 3.88 (3H,s) , 3.95 (3H,s) , 7.44 (1 H, dd, J=2.0, 8.8Hz) , 7.71 (1H, d, J=8.8Hz), 7.74 (1H, d, J=2.0Hz), 7. 86 (1H, d, J=8.8Hz), 8.11 (1H, dd, J=2.0, 8.8Hz), 8.63 (1H, d, J=2.0Hz ) .
IR νₘₐₓ (KBr) : 3440, 3140, 2954, 1724, 1693, 1608, 1500, 1439, 1331, 1 246,1165,1119 cm⁻¹.

### Example 7

### Methyl 4-(5-chloro-3-methylbenzo[b]thiophene-2-sulfonylamino)-3-methoxybenzoate

Into 4 mL of pyridine were dissolved 120 mg of methyl 4-amino-3-methoxybenzoate and 150 mg of 5-chloro-2-chlorosulfonyl-3-methylbenzo[b]thiophene, followed by 14 hours of stirring at room temperature. After confirmation of disappearance of the starting material, the reaction was terminated by adding water at 0°C, followed by extraction with ethyl acetate. The organic layer was washed with saturated brine and then dried over anhydrous sodium sulfate. The solvent was removed by evaporation and then the residue was purified by silica gel chromatography (chloroform/methanol = 9/1) to obtain 110 mg of the title compound as colorless amorphous.
¹H-NMR (CDCl₃) δ 2.55 (3H, s), 3.79 (3H, s), 3.86 (3H, s), 7.42 (1 H, dd, J=2.0,8.6Hz), 7.45 (1H, dd, J=2.0,8.6 Hz) , 7.61 (1H, d, J=2.0H z), 7.62 (1H, d, J=8.6Hz), 7.68 (1H, d, J=8.6Hz), 7.71 (1H, d, J=2.0H z).
IR νₘₐₓ (KBr) : 3248, 2951, 1716, 1601, 1512, 1439, 1350, 1284, 1242, 1 161,1115 cm⁻¹.

### Example 8

### Methyl 4-(5-chloro-3-methylbenzo[b]thiophene-2-sulfonylamino)-3-nitrobenzoate

In the same manner as in Example 6, 146 mg of the title compound was obtained as yellow powder from 122 mg of methyl 4-amino-3-nitrobenzoate.
Melting point: 164-165°C
¹H-NMR (CDCl₃) : δ 2,47 (3H, s), 3.84 (3H,s), 7.56 (1H, d, J=8.6Hz ) , 7.57 (1H, brd, J=8.6Hz), 8.01 (1H, d, J=1.8Hz), 8.06 (1H, d, J=8.6 Hz) , 8.07 (1H, brd, J=8.6Hz) , 8.25 (1H, d, J=1.8Hz).
IR νₘₐₓ (KBr) : 3442, 3237, 3060, 2949, 1732, 1621, 1535, 1507, 1440, 1 356,1297,1164,1106 cm⁻¹.

### Example 9

### 5-chloro-3-methylbenzo[b]thiophene-2-sulfonic acid (2,4-dimethanesulfonylphenyl)amide

In the same manner as in Example 6, 368 mg of the title compound was obtained as colorless powder from 200 mg of 2,4-dimethanesulfonylaniline.
Melting point: 176-178°C
¹H-NMR (DMSO-d₆) : δ 2,65 (3H,s) , 3.00 (3H,s) , 3.07 (3H,s) , 7.44 (1H, dd, J=1.8,8.6Hz) , 7.71 (1H, d, J=8.6Hz) , 7.76 (1H, d, J=1.8Hz) ,7.92 (1H, d, J=8.8Hz) , 8.04 (1H, dd, J=1.8,8.8Hz), 8.34 (1H, d, J=1. 8Hz) .
IR νₘₐₓ (KBr) : 3236, 3020, 1593, 1489, 1392, 1354, 1304, 1157 cm⁻¹.

### Example 10

### 5-chloro-3-methylbenzo[b]thiophene-2-sulfonic acid (4-acetyl-2-nitrophenyl)amide

In the same manner as in Example 6, 59 mg of the title compound was obtained as colorless powder from 96 mg of 4-acetyl-2-nitroaniline.
Melting point: 130-131°C
¹H-NMR (CDCl₃): δ 2.58 (3H, s), 2.69 (3H, s) , 7.46 (1H, dd, J=2.0,8 .6Hz) ,7.73 (1H, d, J=8.6Hz), 7.78 (1H, d, J=2.0Hz) ,8.05 (1H, d, J=8 .8Hz) ,8.16 (1H, dd, J=1.8,8.8 Hz), 8.74 (1H, d, J=1.8Hz).
IR νₘₐₓ (KBr) : 3745, 3479, 3363, 3262, 3089, 2927, 2858, 1689, 1620, 1 531, 1419, 1354, 1115, 1080 cm⁻¹.

### Example 11

### 5-chloro-3-methylbenzo[b]thiophene-2-sulfonic acid (4-acetyl-2-methanesulfonylphenyl)amide

Into a mixed solvent of 20 mL of THF and 5 mL of DMF was dissolved 241 mg of 4-amino-3-methanesulfonylacetophenone, followed by addition of 136 mg of sodium hydride (oily, 60%) at -78°C. After 20 minutes of stirring at the same temperature, 350 mg of 5-chloro-2-chlorosulfonyl-3-methylbenzo[b]thiophene was added at -78°C, and the mixture was gradually warmed and stirred at -10°C for 1 hour. After confirmation of disappearance of the starting material, the reaction was terminated by adding saturated aqueous ammonium chloride solution at 0°C, followed by extraction with ethyl acetate. The organic layer was washed with saturated brine and then dried over anhydrous sodium sulfate. The solvent was removed by evaporation and the residue was purified by silica gel chromatography (ethyl acetate/hexane = 1/1) to obtain 427 mg of the title compound as colorless powder. Melting point: 207-209°C
¹H-NMR (CDCl₃) : δ 2.56 (3H, s), 2.69 (3H, s), 3.07 (3H, s), 7.46 ( 1H, dd, J=1.9, 8.7Hz) ,7.72-7.79 (2H,m) , 7.86 (1H, d, J=8.6Hz), 8.10 (1H, d, J=8.6Hz), 8.40 (1H, d, J=1.9Hz).
IR νₘₐₓ (KBr) : 3456, 3236, 3086, 3005, 2924, 2854, 1670, 1593, 1489, 1 389, 1354, 1308, 1261, 1165, 1130, 1053 cm⁻¹.

### Example 12

### 5-chloro-3-methylbenzo[b]thiophene-2-sulfonic acid (4-benzoyl-2-methanesulfonylphenyl)amide

In the same manner as in Example 11, 68 mg of the title compound was obtained as colorless powder from 94 mg of 4-amino-3-methanesulfonylbenzophenone.
Melting point: 144-146°C
¹H-NMR (CDCl₃) : δ 2.70 (3H, s), 3.08 (3H, s), 7.45-7.50 (3H, m), 7 .58-7.62 (2H, m), 7.68-7.71 (4H, m) ,7.85 (1H, d, J=8.6Hz),7.97 (1H , d, J=8.6Hz), 8.31 (1H,brs).
IR νₘₐₓ (KBr) :3456, 3248, 3001, 2927, 2858, 2256, 1709, 1655, 1597, 1 496, 1450, 1389, 1350, 1308, 1161, 1130, 1084 cm⁻¹.

### Example 13

### 5-chloro-3-methylbenzo[b]thiophene-2-sulfonic acid (4-hydroxymethyl-2-methanesulfonylphenyl)amide

Into 10 mL of toluene was dissolved 305 mg of the compound of Example 1, and the solution was cooled to -78°C, followed by addition of 2.2 mL of 1.01 M toluene solution of diisobutylaluminum hydride. After 20 minutes of stirring at the same temperature, the mixture was gradually warmed to 0°C and stirred for 1 hour. After the reaction was terminated by adding water, the mixture was diluted with ethyl acetate and saturated aqueous potassium sodium tartrate solution were added, followed by 30 minutes of stirring at room temperature. The mixture was extracted with ethyl acetate and the organic layer was washed with saturated brine and then dried over anhydrous sodium sulfate. The solvent was removed by evaporation and the residue was purified by silica gel chromatography (ethyl acetate/hexane = 1/1) to obtain 230 mg of the title compound as colorless powder.
Melting point: 183-184°C
¹H-NMR (CDCl₃) :δ 1.83 (1H, brs), 2.69 (3H,s), 2.97 (3H, s), 4.69 (2H, d, J=5.7Hz), 7.47 (1H, dd, J=2.1Hz, 8.7Hz) , 7.57 (1H, dd, J=2.1H z, 8.7Hz) , 7.74 (1H, d, J=9.3Hz) , 7.78 (1H, d, J=9.3Hz), 7.79 (1H, d, J=2.1Hz), 7.86 (1H, d, J=2.1Hz), 9.49 (1H, brs).
IR νₘₐₓ (KBr) : 3563, 3236, 1612, 1500, 1392, 1277, 1142cm⁻¹.

### Example 14

### Ethyl 4-(5-chloro-3-methylbenzo[b]thiophene-2-sulfonylamino)benzoate

Into 3 mL of pyridine was dissolved 60 mg of ethyl 4-aminobenzoate, and 123 mg of 5-chloro-2-chlorosulfonyl-3-methylbenzo[b]thiophene was added at 0°C, followed by 2 hours of stirring at room temperature. After confirmation of disappearance of the starting material, 2 mol/L hydrochloric acid was added, followed by extraction with ether. The organic layer was washed with saturated brine and then dried over anhydrous magnesium sulfate. The solvent was removed by evaporation under reduced pressure and the resulting crude product was purified by silica gel column chromatography (ethyl acetate/hexane = 1/3) to obtain 80 mg of the title compound as light pink powder.
Melting point: 224-226°C
¹H-NMR (DMSO-d₆) δ 1.26 (3H, t, J=7.1Hz), 2.50 (3H, s), 4.23 (2H, q, J=7.1Hz), 7.27 (2H,d,J=8.8Hz) ,7.57 (1H, dd, J=2.0,8.6Hz), 7.84 (2H, d, J=8.8Hz), 8.01 (1H, d, J=2.0Hz), 8.05 (1H, d, J=8.6Hz). IR νₘₐₓ (KBr) : 3213, 1696, 1608, 1511, 1347, 1288, 1159 cm⁻¹.

### Example 15

### 5-chloro-3-methylbenzo[b]thiophene-2-sulfonic acid (4-benzoylphenyl)amide

In the same manner as in Example 14, 187 mg of the title compound was obtained as colorless powder from 126 mg of 4-benzoylaniline.
Melting point: 198-200°C
¹H-NMR (CDCl₃) : δ 2.56 (3H,s) , 7.22-7.26 (2H,m) , 7.44-7.48 (3H, m), 7.55-7.60 (1H, m), 7.70-7.76 (6H,m) .
IR νₘₐₓ (KBr) : 3213, 2927, 1724, 1639, 1589, 1508, 1450, 1408, 1288, 1 234, 1149 cm⁻¹.

### Example 16

### 5-chloro-3-methylbenzo[b]thiophene-2-sulfonic acid (2-methanesulfonylphenyl)amide

In the same manner as in Example 14, 52 mg of the title compound was obtained as colorless powder from 100 mg of 2-methanesulfonylaniline.
Melting point: 191-193°C
¹H-NMR (CDCl₃) : δ 2.68 (3H,s) , 3.00 (3H,s), 7.24-7.29 (1H, m), 7 .35 (1H, s) ,7.74-7.80 (2H,m), 7.46 (1H, dd, J=1.8,8.6Hz), 7.74-7. 80 (1H, m), 7.85 (1H, dd, J=1.5,7.9Hz)
IR νₘₐₓ (KBr) :3467, 3371, 3228, 3016, 2927, 2858, 1712, 1624, 1566, 1 485, 1408, 1288, 1134, 1026 cm⁻¹.

### Example 17

### Methyl 4-(5-fluoro-3-methylbenzo[b]thiophene-2-sulfonylamino)-3-methanesulfonylbenzoate

Into 300 mL of THF was dissolved 14.0g of methyl 4-amino-3-methanesulfonylbenzoate, followed by addition of 6.10 g of sodium hydride (oily, 60%) at 0°C. After 40 minutes of stirring at the same temperature, 16.0 g of 5-fluoro-2-chlorosulfonyl-3-methylbenzo[b]thiophene was added at 0°C, followed by 3 hours of stirring at room temperature. After confirmation of disappearance of the starting material, the reaction was terminated by adding 2 mol/L hydrochloric acid at 0°C, followed by extraction with ethyl acetate. The organic layer was washed with saturated aqueous sodium hydrogen carbonate solution and saturated brine and then dried over anhydrous sodium sulfate. The solvent was removed by evaporation and the residue was diluted with ethyl acetate. After the solution was treated with active carbon, purification by recrystallization (ethyl acetate/ether) afforded 24.8 g of the title compound as colorless powder. Melting point: 202-204°C
¹H-NMR (CDCl₃) : δ 2. 69 (3H, s), 3.06 (3H, s), 3. 90 (3H, s), 7.28 (1 H, ddd, J=2.6, 8.7, 8.9Hz), 7.46 (1H, dd, J=2.6,9.2Hz), 7.76 (1H, dd, J =4.7,8.9Hz), 7.87 (1H, d, J=8.8Hz), 8.19 (1H, dd, J=2.0,8.8Hz), 8.5 0 (1H, d, J=2.0Hz), 9.83 (1H, s). IR νₘₐₓ (KBr): 3182, 1724, 1604, 1504, 1442, 1396, 1346, 1303, 1157 cm⁻¹.

### Example 18

### Methyl 4-(5-methyl-3-methylbenzo[b]thiophene-2-sulfonylamino)-3-methanesulfonylbenzoate

Into 8.0 mL of THF was dissolved 183 mg of methyl 4-amino-3-methanesulfonylbenzoate, followed by addition of 96 mg of sodium hydride (oily, 60%) at 0°C. After 20 minutes of stirring at the same temperature, 250 mg of 5-methyl-2-chlorosulfonyl-3-methylbenzo[b]thiophene was added at 0°C, followed by 6 hours of stirring at room temperature. After confirmation of disappearance of the starting material, the reaction was terminated by adding 1 mol/L hydrochloric acid at 0°C, followed by extraction with chloroform. The organic layer was washed with saturated brine and then dried over anhydrous sodium sulfate. The solvent was removed by evaporation and then the residue was purified by silica gel column chromatography (ethyl acetate/n-hexane = 3/1 to 1/1) to obtain 181 mg of the title compound as colorless powder. Melting point: 179-181°C
¹H-NMR (CDCl₃) : δ 2.48 (3H,s), 2.70 (3H, s) , 3.02 (3H,s), 3.89 (3 H, s), 7.35 (1H, dd, J=2.2, 8.8Hz), 7.60 (1H, d, J=2.2Hz), 7.69 (1H, d , J=8.8Hz), 7.88 (1H, d, J=8.8Hz) , 8.18 (1H, dd, J=1.8,8.8Hz) , 8.50 (1H, d, J=1.8Hz).
IR νₘₐₓ (KBr) :3460, 3178, 3016, 2927, 2861, 1724, 1604, 1500, 1439, 1396, 1300, 1130, 1061 cm⁻¹.

### Example 19

### 5-fluoro-3-methylbenzo[b]thiophene-2-sulfonic acid (4-acetyl-2-methanesulfonylphenyl)amide

Into a mixed solvent of 168 mL of THF and 42 mL of DMF was dissolved 6.30 g of (4-amino-3-methanesulfonyl)acetophenone, followed by addition of 4.70 g of sodium hydride (oily, 60%) at -40°C. After 10 minutes of stirring at the same temperature, 8.60 g of 5-fluoro-2-chlorosulfonyl-3-methylbenzo[b]thiophene was added at the same temperature, followed by 4 hours of stirring at the same temperature. After confirmation of disappearance of the starting material, the reaction was terminated by adding 1 mol/L hydrochloric acid at the same temperature and then the mixture was rendered pH 1 with concentrated hydrochloric acid, followed by extraction with chloroform. The organic layer was washed with saturated brine and then dried over anhydrous sodium sulfate. The solvent was removed by evaporation and then the residue was diluted with chloroform. After the solution was treated with active carbon, the solvent was removed by evaporation and the resulting crystals were washed with methanol to obtain 10.9 g of the title compound as colorless powder.
Melting point: 174-175°C ¹H-NMR (CDCl₃) : δ 2.56 (3H, s), 2.69 (3H, s), 3.08 (3H, s), 7.29 (1 H,ddd,J=2.5, 8.8, 8.8Hz), 7.47 (1H, dd, J=2.5, 8.8Hz), 7.77 (1H, dd, J =4.6, 8.8Hz), 7.84 (1H, d, J=8.6Hz), 8.12 (1H, dd, J=2.2, 8.6Hz), 8.4 2 (1H, d, J=2.2Hz), 9.83 (1H, brs).
IR νₘₐₓ (KBr) : 3243, 3092, 3006, 2925, 1672, 1599, 1443, 1392, 1262, 1130, 1056, 1029 cm⁻¹.

### Example 20

### Methyl 4-(3-methylbenzo[b]thiophene-2-sulfonylamino)-3-methanesulfonylbenzoate

Into a mixed solvent of 30 mL of methanol and 30 mL of dioxane was dissolved 640 mg of 5% palladium/carbon, followed by 10 minutes of stirring under a hydrogen atmosphere. Under an argon atmosphere, 330 mg of methyl 4-(5-chloro-3-methylbenzo[b]thiophene-2-sulfonylamino)-3-methanesulfonylbenzoate was added, followed by 3 days of stirring under a hydrogen atmosphere at 5 atm. After confirmation of disappearance of the starting material, the reaction mixture was filtered and purification by silica gel column chromatography (ethyl acetate/n-hexane = 2/1) to obtain 70 mg of the title compound as colorless powder. Melting point: 170-172°C
¹H-NMR (CDCl₃) : δ 2.75 (3H, s), 3.04 (3H,s) , 3.90 (3H,s) , 7.49 (1 H, dd, J=7.1, 7.7Hz) , 7.51 (1H, dd, J=7.1, 7.7Hz), 7.83 (2H, d, J=7.7Hz ), 7.89 (1H, d, J=8.8Hz), 8.20 (1H, dd, J=2.0, 8.8Hz), 8.51 (1H, d, J =2.0Hz) ,9.82 (1H,s).
IR νₘₐₓ (KBr) :3209, 1720, 1604, 1500, 1442, 1392, 1350, 1308, 1165, 1122 cm⁻¹.

### Example 21

### Methyl (2S)-2-[4-(5-fluoro-3-methylbenzo[b]thiophene-2-sulfonylamino)-3-methanesulfonyl]benzoylamino-3-hydroxypropionate

Into 450 mL of chloroform was dissolved 14.3 g of [4-(5-fluoro-3-methylbenzo[b]thiophene-2-sulfonylamino)-3-methanesulfonyl]benzoic acid obtained by hydrolysis of 24.8 g of methyl 4-(5-fluoro-3-methylbenzo[b]thiophene-2-sulfonylamino)-3-methanesulfonylbenzoate. After 7.54 g of L-serine methyl ester hydrochloride and 9.30 g of EDC hydrochloride were added thereto at room temperature, 6.80 mL of triethylamine was added at 0°C. After 2 hours of stirring at the same temperature, the reaction was terminated by adding 2 mol/L hydrochloric acid at 0°C and the mixture was extracted with chloroform. The organic layer was washed with saturated brine and then dried over anhydrous sodium sulfate. The solvent was removed by evaporation and the residue was purified by silica gel chromatography (ethyl acetate) to obtain 12.9 g of the title compound as colorless amorphous. ¹H-NMR (CDCl₃) : δ 2.71 (3H, s), 3.07 (3H,s) , 3.81 (3H, s), 4.00 (1 H, dd, J=4.2, 11.4Hz) , 4.15 (1H, dd, J=5.4, 11.4Hz), 4.85 (1H, dd, J=4. 2,5.4Hz), 7.33 (1H, dd, J=2.1, 8.6Hz), 7.48 (1H, dd, J =2.1, 8.6Hz), 7 .79 (1H, dd, J=4.6, 8.6Hz), 7.87 (1H, d, J=8.8Hz), 8.00 (1H, dd, J=2.1 ,8.8Hz), 8.32 (1H, d, J=2.1Hz), 9.76 (1H, s).
IR νₘₐₓ (KBr) : 3401, 1735, 1655, 1606, 1510, 1491, 1440, 1353, 1308, 1 164, 1136cm⁻¹.

### Example 22

### Methyl 2-[4-(5-fluoro-3-methylbenzo[b]thiophene-2-sulfonylamino)-3-methanesulfonylphenyl]-4,5-dihydro-oxazole-4-carboxylate

Into 180 mL of THF was dissolved 12.9 g of methyl (2S)-2-[4-(5-fluoro-3-methylbenzo[b]thiophene-2-sulfonylamino)-3-methanesulfonyl]benzoylamino-3-hydroxy-propionate, and 6.80 g of Burgess reagent (J. Org. Chem., 38, 26, (1973); J. Org. Chem., 58, 4494 (1993)) was added, followed by 2 hours of stirring at 60°C. After confirmation of disappearance of the starting material, the solvent was removed by evaporation and water was added, followed by extraction with ethyl acetate. The organic layer was washed with saturated brine and then dried over anhydrous sodium sulfate. The solvent was removed by evaporation and the residue was purified by silica gel chromatography (ethyl acetate/hexane = 9/1) to obtain 9.92 g of the title compound as colorless amorphous.
¹H-NMR (CDCl₃) : δ 2.68 (3H, s), 3.02 (3H, s), 3.81 (3H, s), 4.60 ( 1H, dd, J=9.0, 10.6Hz) , 4.69 (1H, dd, J=7.9, 9.0Hz), 4.93 (1H, dd, J=7. 9, 10.6Hz), 7.29 (1H, ddd, J=2.1, 8.8, 8.8Hz), 7.46 (1H, dd, J=2.1, 8.8 Hz), 7.76 (1H, dd, J=4.6, 8.8Hz), 7.87 (1H, d, J=8.8Hz), 8.15 (1H, dd , J=2.1, 8.8Hz) , 8.43 (1H, d, J=2.1Hz), 9.81 (1H, s).
IR νₘₐₓ (KBr) : 3226, 1737, 1647, 1608, 1498, 1441, 1395, 1355, 1308, 1 248, 1211, 1164 cm⁻¹.

### Example 23

### Methyl 2-[4-(5-fluoro-3-methylbenzo[b]thiophene-2-sulfonylamino)-3-methanesulfonylphenyl]oxazole-4-carboxylate

Into 40 mL of dichloromethane was dissolved 1.10 g of methyl 2-[4-(5-fluoro-3-methylbenzo[b]thiophene-2-sulfonylamino)-3-methanesulfonylphenyl]-4,5-dihydro-oxazole-4-carboxylate, followed by addition of 498 mg of bromotrichloromethane at -20°C. Further, 700 mg of DBU was added dropwise at the same temperature and the whole was stirred at the same temperature for 5 minutes and then warmed to 0°C, followed by 3.5 hours of stirring. After confirmation of disappearance of the starting material, the reaction was terminated by adding saturated aqueous sodium hydrogen carbonate solution at 0°C, followed by extraction with ethyl acetate. The organic layer was washed with saturated brine and then dried over anhydrous magnesium sulfate. The solvent was removed by evaporation and the residue was purified by silica gel chromatography (ethyl acetate/methanol = 20/1) to obtain 597 mg of the title compound as colorless powder.
Melting point: 290-292°C
¹H-NMR (CDCl₃) : δ 2.70 (3H, s), 3.06 (3H, s), 3.95 (3H, s), 7.30 (1 H, ddd, J=2.4, 8.7, 8.7Hz), 7.47 (1H, dd, J=2.4, 9.0Hz), 7.77 (1H, dd, J =4.8, 9.0Hz), 7.94 (1H, d, J=9.0Hz), 8.27 (1H, s), 8.28 (1H, dd, J=2 .1, 9.0Hz), 8.57 (1H, d, J=2.1Hz), 9.78 (1H, s).
IR νₘₐₓ (KBr) : 3243, 1720, 1618, 1590, 1518, 1485, 1440, 1355, 1320, 1 303, 1259, 1162, 1136 cm⁻¹.

### Example 24

### 2-[4-(5-fluoro-3-methylbenzo[b]thiophene-2-sulfonylamino)-3-methanesulfonylphenyl]oxazole-4-carboxylic acid

Into 150 mL of methanol was dissolved 7.85 g of methyl 2-[4-(5-fluoro-3-methylbenzo[b]thiophene-2-sulfonylamino)-3-methanesulfonylphenyl]oxazole-4-carboxylate, and 15 mL of 10% aqueous sodium hydroxide solution and 15 mL of water were added thereto at room temperature, followed by 15 minutes of stirring. The precipitated crystals were dissolved by adding 90 mL of water, and the solution was stirred at the same temperature for 17 hours. After the solvent was removed by evaporation, 45 mL of 1 mol/L hydrochloric acid was added to the residue, the precipitated crystals were collected by filtration and washed with water, and 100 mL of DMF was added to the resulting crude crystals, followed by heating under refluxing. After filtration at a hot state, 70 mL of ethanol was added and recrystallization was carried out. The resulting crystals were collected by filtration, washed several times with ethanol and water alternatively, and dried over diphosphorus pentoxide under reduced pressure to obtain 5.78 g of the title compound as colorless powder.
Melting point: 289-291°C
¹H-NMR (DMSO-d₆) : δ 2.55 (3H, s), 3.41 (3H, s), 7.44 (1H, ddd, J=1. 8, 8.7, 9.0Hz), 7.64 (1H, d, J=8.4Hz), 7.79 (1H, dd, J=1.8, 9.9Hz), 8. 08 (1H, dd, J=4.8, 8.7Hz), 8.19 (1H, dd, J=1.8, 8.9Hz), 8.41 (1H, d, J= 1.8Hz), 8.84 (1H, s).
IR νₘₐₓ (KBr) : 3232, 1717, 1690, 1616, 1487, 1440, 1355, 1313, 1161, 1 140 cm⁻¹.

### Example 25

### Methyl 2-[4-(5-chloro-3-methylbenzo[b]thiophene-2-sulfonylamino)-3-methanesulfonylphenyl]oxazole-4-carboxylate

Into 10 mL of dichloromethane was dissolved 495 mg of copper dibromide, followed by addition of 310 mg of hexamethyltetramine at room temperature. Further, 337 mg of DBU was added dropwise at 0°C, the whole was stirred at the same temperature for 5 minutes and then methyl 2-[4-(5-chloro-3-methylbenzo[b]thiophene-2-sulfonylamino)-3-methanesulfonylphenyl]-4,5-dihydro-oxazole-4-carboxylate was added at 0°C, followed by 3 hours of stirring at room temperature. After confirmation of disappearance of the starting material, ethyl acetate was added to the reaction mixture. The organic layer was washed with a 1:1 mixed solution of saturated aqueous ammonium chloride solution and 25% aqueous ammonia solution, saturated aqueous sodium hydrogen carbonate and saturated brine and then dried over anhydrous magnesium sulfate. The solvent was removed by evaporation and the residue was purified by silica gel chromatography (chloroform/methanol = 10/1) to obtain 110 mg of the title compound as colorless powder.
Melting point: 237-239°C
¹H-NMR (CDCl₃) : δ 2.70 (3H, s), 3.05 (3H, s), 3.95 (3H, s), 7.47 (1 H, dd, J=2.1, 8.7Hz), 7.74 (1H, d, J=8.7 Hz), 7.79 (1H, d, J=2.1Hz), 7 .93 (1H, d, J=9.0Hz), 8.27 (1H, s), 8.28 (1H, dd, J=2.1, 9.0Hz), 8.56 (1H, d, J=2.1Hz), 9.72 (1H, brs).
IR νₘₐₓ (KBr) : 3231, 1744, 1486, 1317, 1136 cm⁻¹.

### Example 26

### 2-[4-(5-chloro-3-methylbenzo[b]thiophene-2-sulfonylamino)-3-methanesulfonylphenyl]oxazole-4-carboxylic acid

In the same manner as in Example 24, 68 mg of the title compound was obtained as colorless powder from 70 mg of methyl 2-[4-(5-chloro-3-methylbenzo[b]thiophene-2-sulfonylamino)-3-methanesulfonylphenyl]oxazole-4-carboxylate. Melting point: 296-298°C
¹H-NMR (DMSO-d₆) :δ 2.58 (3H, s), 3.37 (3H, s), 7.55 (1H, dd, J=2.1 , 8.7Hz), 7.62 (1H, d, J=8.7Hz), 8.01 (1H, d, J=2.1Hz), 8.09 (1H, d, J=8.4Hz), 8.12 (1H, dd, J=2.1, 8.4Hz), 8.40 (1H, d, J=2.1Hz), 8.83 ( 1H, s)
IR νₘₐₓ (KBr) : 3221, 2924, 1701, 1485, 1311, 1153 cm⁻¹.

### Example 27

### Disodium 2-[4-(5-fluoro-3-methylbenzo[b]thiophene-2-sulfonylamino)-3-methanesulfonylphenyl]oxazole-4-carboxylate

Into 30 mL of methanol was dissolved 290 mg of methyl 2-[4-(5-fluoro-3-methylbenzo[b]thiophene-2-sulfonylamino)-3-methanesulfonylphenyl]oxazole-4-carboxylate, followed by addition of 77 mg of sodium methoxide. After 8 hours of stirring at room temperature, ether was added and the precipitated crystals were collected by filtration and washed with ether to obtain 300 mg of the title compound as colorless powder.
Melting point: 341-343°C
¹H-NMR (DMSO-d₆) : δ 2.49 (3H, s), 3.42 (3H,s), 7.26 (1H, ddd, J=2. 4,8.8, 9.0Hz), 7.40 (1H, dd, J=9.0,9.0Hz), 7.56 (1H, dd, J=2.4, 8.9Hz ), 7.89 (1H, dd, J=2.2, 9.0Hz), 7.95 (1H, d, J=9.0Hz), 8.19 (1H, d, J=2 .2Hz).
IR νₘₐₓ (KBr) : 3490, 1609, 1570, 1523, 1470, 1441, 1400, 1302, 1280, 1 119 cm⁻¹.

In the following, Compounds of Examples 28 to 49 were synthesized in the same manner as in Example 1.

**Table 1**

| | | | | | |
|---|---|---|---|---|---|
| 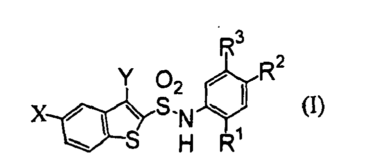 | | | | | |

| Example | X | Y | R¹ | R² | R³ |
|---|---|---|---|---|---|
| 28 | Cl | Me | H | NO₂ | H |
| 29 | Cl | Me | H | CN | H |
| 30 | Cl | Me | H | COMe | H |
| 31 | Cl | Me | H | CONH₂ | H |
| 32 | Cl | Me | H | COCH₂SCH₂CO₂Me | H |
| 33 | Cl | Me | OMe | NO₂ | H |
| 34 | Cl | Me | NO₂ | CN | H |
| 35 | Cl | Me | NO₂ | NO₂ | H |
| 36 | Cl | Me | NO₂ | OMe | H |
| 37 | Cl | Me | OMe | CONHCH₂CO₂Et | H |
| 38 | Cl | Me | CO₂Me | OMe | OMe |
| 39 | Cl | Me | H | SO₂(CH₂)₂CH₃ | H |
| 40 | H | Me | H | SO₂(CH₂)₂CH₃ | H |
| 41 | Me | Me | H | SO₂(CH₂)₂CH₃ | H |
| 42 | Cl | Me | SO₂Me | CO₂CH(CH₃)₂ | H |
| 43 | Cl | Me | SO₂Me | CONHCH₂CO₂Et | H |
| 44 | Cl | Me | SO₂Me | 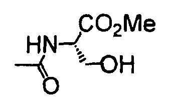 | H |
| 45 | Cl | Me | SO₂Me | 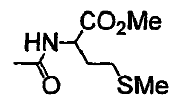 | H |
| 46 | Cl | Me | SO₂Me | 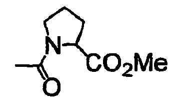 | H |
| 47 | Cl | Me | SO₂Me | 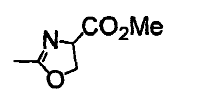 | H |
| 48 | F | Me | SO₂Me | 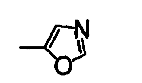 | H |
| 49 | F | Me | SO₂NEt₂ | CO₂Me | H |

### Example 50

### 2-[4-(5-chloro-3-methylbenzo[b]thiophene-2-sulfonylamino)-3-methoxybenzoylamino]acetic acid

Into 25 mL of ethanol was dissolved 104 mg of the compound of Example 26, and 1 mL of 1 N aqueous sodium hydroxide solution was added at room temperature, followed by 15 hours of stirring at the same temperature. After confirmation of disappearance of the starting material, the solvent was removed by evaporation, followed by extraction with ether. After 2 mol/L hydrochloric acid was added to the aqueous layer, the mixture was extracted with ethyl acetate. The organic layer was washed with saturated brine and then -dried over anhydrous sodium sulfate. The solvent was removed by evaporation and the resulting powder was washed with ether to obtain 97 mg of the title compound as light yellow powder. Melting point: 282-285°C
¹H-NMR (CDCl₃/CD₃OD) : δ 2.50 (3H, s), 3.82 (2H, s), 3.84 (3H, s), 7 .10-7.15 (2H, m), 7.20-7.35 (2H,m), 7.60-7.70 (2H, m).
IR νₘₐₓ (KBr) :3394, 2974, 1604, 1554, 1493, 1412, 1284, 1230, 1130 c m⁻¹.

### Example 51

### Sodium methyl 4-(5-chloro-3-methylbenzo[b]thiophene-2-sulfonylamino)-3-methanesulfonylbenzoate

Into 8 mL of THF was dissolved 115 mg of the compound of Example 1, followed by addition of 15 mg of sodium hydride (oily, 60%) at room temperature. After 1.5 hours of stirring at the same temperature, the solvent was removed by evaporation and the resulting powder was washed with ether to obtain 61 mg of the title compound as colorless powder. Melting point: >300°C
¹H-NMR (DMSO-d₆) : δ 2.51 (3H, s), 3.37 (3H, s), 3.72 (3H, s), 7.39 (1H, d, J=2.1, 8.8Hz), 7.40 (1H, dd, J=1.8, 8.5Hz), 7.68 (1H, dd, J=1. 8,8.8Hz), 7.80 (1H, d, J=1.8Hz), 7.93 (1H, d, J=8.5Hz), 8.23 (1H, d, J=1.8Hz).
IR νₘₐₓ (KBr) :3448, 1705, 1597, 1481, 1442, 1292, 1134, 1103 cm⁻¹.

After the compounds of Examples 43, 44 and 46 were subjected to ester hydrolysis, the products were formed into sodium salts in the same conditions as in Example 51 to synthesize compounds of Examples 52, 53 and 54.

**Table 2**

| | | | | | |
|---|---|---|---|---|---|
| 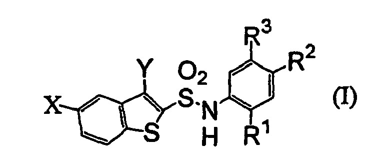 | | | | | |

| Example | X | Y | R¹ | R² | R³ |
|---|---|---|---|---|---|
| 52 | Cl | Me | SO₂Me | CONHCH₂CO₂Na | H |
| 53 | Cl | Me | SO₂Me | 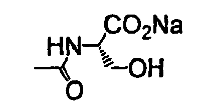 | H |
| 54 | Cl | Me | SO₂Me | 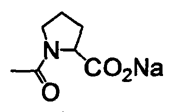 | H |

The following show instrumental data in each Examples.

**Table 3**

| Example | Melting point (°C) | H¹-NMR (δ) | | IR (ν cm⁻¹, KBr) |
|---|---|---|---|---|
| 28 | Amorphous | CDCl₃ | 2.62 (3H, s), 7.29 (2H, d, J 9.1Hz), 7.46 (1H, dd, J=2. 0, 8.7Hz), 7.47 (1H, brs), 7.73 (1H, d, J=8.7Hz), 7.7 6 (1H, d, J=2.0Hz), 8.15 (2 H,d,J= 9.1Hz) | 3248, 3084, 2925, 2856, 1596, 1521, 1342, 1160, 1113 |
| 29 | 226-227 | CDCl₃/ CD₃OD | 2.60(3H,s),7.29(2H,d,J 9.0Hz), 7.45 (1H, dd, J=2. 1, 8.7Hz), 7.53 (2H, d, J=9 .0Hz), 7.75 (1H, d, J=2.1H z), 7.76-7.77 (1H, m) | 3236, 2222, 1606, 1508, 1469, 1356, 1160 |
| 30 | 223-225 | CDCl₃ | 2.53 (3H, s), 2.54 (3H, s), 7.20-7.23 (2H, m), 7.45 (1H,dd, J=2.1, 8.6Hz), 7.72 (2H, d ,J=8.6Hz), 7.88 (2H,d,8. 6Hz) | 3178, 2927, 2233, 1666, 1593, 1508, 1404, 1338, 1273, 1153 |
| 31 | 254-258 | CDCl₃ | 2.55(3H,s),7.24(2H,d,J = 7.4Hz),7.36-7.45(3H,m), 7.72 (2H, d, J=8.6Hz) | 3379, 3255, 3174, 2911, 2846, 2765, 1651, 1512, 1404, 1335, 1223, 1157 |
| 32 | 151-153 | CDCl₃ | 2.57(3H,s),3.30(2H,s), 3.71 (3H, s), 3.93 (2H, s), 7.20-7.25 (2H, m), 7.44-7.47 (1H, m), 7.71-7.75(2H,m),7.86-7.90(2H,m) | 3221, 3059, 2935, 1736, 1662, 1593, 1512, 1466, 1404, 1338, 1296, 1153 |
| 33 | 188-190 | CDCl₃ | 2.63 (3H, s), 3.92 (3H, s), 7.45 (1H, dd, J=2.0, 8.8Hz ), 7.63 (1H, brs), 7.67 (1H , d, J=2.4Hz), 7.71 (1H, d, J=8.8 Hz), 7.76 (1H, d, J=2.0Hz) 7.84 (1H, dd, J=2.4, 8.8Hz ) | 3293, 3088, 2930, 2846, 1735, 1596, 1525, 1500, 1442, 1402, 1342, 1281, 1254, 1163, 1130 |
| 34 | 204-206 | CDCl₃ | 2.71 (3H, s), 7.51 (1H, dd, J=2.0, 8.6Hz), 7.76 (1H, d , J=8.6Hz), 7.83 (1H, dd, J =2.0, 8.8Hz), 7.85 (1H, d, J=2.0 Hz), 8.12 (1H, d, J=8.8Hz) , 8.51 (1H, d, J=2.0Hz) | 3234, 3081, 2923, 2235, 1620, 1561, 1538, 1499, 1415, 1360, 1324, 1278, 1164, 1145, 1113 |
| 35 | 162-164 | CDCl₃ | 2.71 (3H, s), 7.50 (1H, dd, J=2.0, 8.6Hz), 7.74 (1H, d ,J= 8.6Hz), 7.80 (1H, d, J=2.0 Hz), 8.15 (1H, d, J=9.4Hz) , 8.43 (1H, dd, J=2.5, 9.4Hz ), 9.08 (1H, d, J=2.5Hz) | 3398, 3232, 1604, 1493, 1423, 1346, 1165 |
| 36 | 120-122 | CDCl₃ | 2.52(3H,s),3.82(3H,s), 7.18-7.22 (1H, m), 7.42-7.50(2H,m),7.67-7.73 (2H, m), 7.88 (1H, d, J =9.2Hz) | 3325, 3078, 2931, 2838, 1905, 1619, 1523, 1439, 1346, 1265, 1153 |
| 37 | 196-198 | CDCl₃ | 1.30(3H,t,J-=7.2Hz), 2.55 (3H, s), 3.7 7(3H, s), 4.18 (2H, d, J-=4.9Hz), 4.25 (2H, q, J-=7.2Hz), 7.33 (1H, d, J-=1.8Hz),7.40- 7.45 (2H, m), 7.60- 7.75(3H,m) | 3278, 1743, 1639, 1550, 1508, 1408, 1350, 1215, 1165 |
| 38 | 203-205 | CDCl₃ | 2.56 (3H, s), 3.82 (6H, s), 3.96 (3H, s), 7.30 (1H, s), 7.41 (1H, s), 7.39- 7.44 (1H, m), 7.67 (1H, s), 7.68-7.75 (1H, m) | 3444, 3170, 2947, 1678, 1612, 1520, 1442, 1362, 1257, 1207, 1161 |
| 39 | 181-183 | CDCl₃ | 0.96 (3H, t, J=7.6Hz), 1.6 9 (2H, sext, J=7.6Hz), 2.5 8 (3H, s), 3.00 (2H, t, J=7. 6Hz), 7.31 (2H, d, J=8.8H z), 7.47 (1H, dd, J=2.1, 8. 7Hz), 7.72 (1H, d, J=8.7Hz ), 7.74 (1H, d, J=2.1Hz), 7 . 79 (2H, d, J=8.8Hz) | 3217, 2966, 1 593, 1493, 14 04, 1350, 128 8, 1238, 1142 , 1088 |
| 40 | 201-203 | CDCl₃ | 0.95 (3H, t, J=7.9Hz), 1.6 6 (2H, sext, J=7.9Hz),2.6 7 (3H, s), 3.05 (2H, t, J=7. 9Hz), 7.40 (2H, d, J=9.0Hz), 7.4 7 (1H, td, J=7.9, 1.5Hz), 7 . 51 (1H, td, J=7.9, 1.5Hz) , 7.74 (2H, d, J=9.0Hz), 7. 84 (2H, dt, J=7.9, 1.5Hz) | 3195, 3058, 2930, 2878, 1594, 1497, 1489, 1346, 1281, 1160, 1132 |
| 41 | 201-203 | CDCl₃ | 0.96 (3H, t, J=7.7Hz), 1.6 9 (2H, sext, J=7.7Hz), 2.4 9(3H, s), 2.59 (3H, s), 3.0 0 (2H, t, J=7.7Hz), 7.11 (1 H,s), 7.30 (2H, d, J=8.6Hz), 7.3 4 (1H, d, J=8.4Hz), 7.56 (1. H, s), 7.68 (1H, d, J=8.4Hz ), 7.78 (2H, d, J=8.6Hz) | 3194, 3059, 2962, 2877, 1593, 1493, 1400, 1342, 1292, 1138 |
| 42 | 164-166 | CDCl₃ | 1.33(6H,d,J=6.2H),2.70 (3H,s),3.06(3H,s),5.22 (1H, q, 6.2Hz), 7.47 (1H, d d, J= 2.1, 8.8Hz), 7.74 (1H, d, J=8.8Hz), 7.79 (1H, d, J =1.5Hz), 7.85 (1H, d, J=8. 6Hz), 8.18 (1H, d, J=8.6Hz), 8.4 8 (1H, d, J=1.5Hz), 9.82 (1 H, s) | 3410, 3244, 2924, 1709, 1604, 1500, 1350, 1304, 1153 |
| 43 | 88-90 | CD₃OD | 1.25 (3H, t, J=7.2Hz), 2.6 4(3H,s),3.19(3H,brs),4 .05 (2H, s), 4.17 (2H, q, J= 7.2Hz) 7.45 (1H, d, J=8.7H z), 7.75 (1H, brd, J=6.9Hz ), 7.85 (1H, d, J=8.7Hz), 7 .87 (1H, s), 7.96 (1H, brd, J=6. 9Hz), 8.34 (1H, d, J=1.8Hz) | 3359, 3232, 3078, 2989, 2927, 1739, 1655, 1604, 1485, 1304, 1211, 1161, 1134 |
| 44 | 154-156 | CD₃OD | 2.65(3H,s),3.07(3H,s), 3.73 (3H, s), 3.89 (1H, dd, J =4.2Hz, 11.4Hz), 3.95 (1H, dd, J=5.4, 11.4Hz), 4.68 (1 H, dd,J=4.2,5.4Hz), 7.47 (1H, dd, J=2.1, 8.4 Hz) ,7.85 (2H, dd, J=2.1, 8.7Hz ), 7.91 (H, d, J=2.1Hz), 8.0 9 (1H, dd, J= 2.1,8.7 Hz), 8.36 (1H, d, J= 2.1Hz) | 3220, 2924, 1736, 1643, 1608, 1496, 1303, 1161, 1130 |
| 45 | Amorphous | CDCl₃ | 2.10 (3H, s), 2.20 (2H, m), 2.56 (2H, m), 2.70 (3H, s), 3.05 (3H, s), 3.78 (3H, s), 4.89 (1H, m), 7.09 (1H, brs), 7.48 (1H, brm), 7.87 (2H, m), 7.96 (1H, m), 8.29 (1H, brs), 9.73 (1H, brs) | 3230, 2921, 2853, 1739, 1653, 1604, 1541, 1492, 1440, 1393, 1353, 1307, 1226, 1166, 1131, 1105 |
| 46 | Amorphous | CDCl₃ | 2.00-2.30 (4H, m), 2.70 (3H, s), 2.98 (3H, s), 3.50-3.65 (2H, m), 3.76 (3H, s), 4.61 (1H, m), 7.48 (1H, d, J=8.4 Hz), 7.75 (1H,d, J=8.4Hz), 7.70-7.80 (3H, m), 8.10 (1H, s), 9.69 (1H, brs) | 3228, 2952, 2925, 1741, 1631, 1496, 1423, 1390, 1353, 1308, 1281, 1203, 1167, 1133, 1107, 1080 |
| 47 | Amorphous | CDCl₃ | 2.69 (3H, s), 3.01 (3H, s), 3.80 (3H, s), 4.58 (1H, dd, J=9.0, 10.5 Hz), 4.68 (1H, dd, J=8.1, 9.0 Hz), 4.93 (1H, dd, J=8.1,10.5 Hz), 7.47 (1H, dd, J=2.1, 8.4 Hz), 7.73 (1H, d, J=8.4 Hz), 7.78 (1H, d, J=2.1 Hz), 7.86 (1H, d, J= 8.7 Hz), 8.14 (1H, dd, J=2.1, 8.7Hz), 8.43 (1H, d, J=2.1 Hz) | 3224, 2958, 1739, 1500, 1308, 1161 |
| 48 | 199-201 | DMSO-d₆ | 2.51(3H, s), 3.17(3H, s), 7.46 (1H, ddd, J=1.8, 9.0, 9.0Hz), 7.52 (1H, d, J=8.4 Hz), 7.75 (1H, d, J= 1.8 Hz), 7.81 (1H, dd, J=1.8, 8.7 Hz), 7.98 (1H, dd, J= 1.8, 8.4Hz), 8.10 (1H, dd, J=4.8, 9.0Hz), 8.15 (1H, s), 8.48 (1H, s) | 3243, 3119, 1606, 1501, 1303, 1151 |
| 49 | 137-140 | CDCl₃ | 1.14 (6H, t, J=7.1 Hz), 2.68 (3H, s), 3.31 (4H, q, J=7.1 Hz), 3.88 (3H, s), 7.29 (1H, ddd, J=2.1, 8.8, 8.8Hz), 7.45 (1H, dd, J=2.1, 8.8 Hz), 7.75 (1H, dd, J=4.8, 8.8 Hz), 7.84 (1H, d, J=8.6 Hz), 8.07 (1H, dd, J=2.0, 8.6 Hz), 8.35 (1H, d, J=2.0 Hz), 9.88 (1H, s) | 3157, 3076, 2979, 2949, 1727, 1604, 1577, 1560, 1521, 1498, 1473, 1458, 1438, 1389, 1344, 1325, 1307, 1280, 1200, 1159, 1134, 1100 |
| 52 | 290-292 | D₂O | 2.50(3H,s),3.47(3H,s), 3.82 (2H, s), 7.22 (1H, d, J= 8.7Hz), 7.42 (1H, dd, J=1.8 ,8.7Hz), 7.69 (1H, dd, J=2. 1, 8.7Hz), 7.80 (1H, d, J=8. 7Hz), 7.83 (1H, d, J=2.1Hz) , 8.21 (1H, d, J=1. 8Hz) | 3410, 1600, 1473, 1400, 1296, 1134, 1107 |
| 53 | 260 | D₂O | 2.47 (3H,s), 3.48 (3H, s), 3.81 (1H, dd, J=5.7Hz, 11.4 Hz), 3.87 (1H, dd, J=3.6, 11.4Hz), 4.38 (1H, dd, J=3. 6,5.7Hz), 7.24 (1H, d, 8.7H z), 7.33 (1H, d, J=8.7Hz), 7 .68-7.73 (3H, m), 8.24 (1H, d, J= 2.1Hz) | 3464, 1597, 1473, 1408, 1292, 1134, 1107 |
| 54 | 298 | D₂O | 1.66-1.78 (2H, m), 2.06-2.12 (2H, m), 2.32 (3H, s), 3.43 (3H, s), 3.37-3.48 (2H, m), 4.14 (1H, m), 6.99 (1H, d, J=8.7 Hz) , 7.10-7.25 (2H, m), 7.34-7.40 (2H, m), 7.51 (1H, d, J=8.7 Hz) 7.95 (1H, d, J=1.8 Hz) | 3419, 1599, 1436, 1308, 1105 |

Next, as for the representative compounds of the present invention, inhibitory activity of chymase and stability in rat plasma were investigated according to the following Test Examples.

### Test Example 1

### Measurement of inhibitory activity of simian chymase

It is known that chmase has a difference in substrate selectivity, depending on the species thereof. It was reported that a primary structure and enzymatic property of Simian chymase are significantly similar to those of human chymase (Miyazaki et al., Kekkan, Vol. 20, p. 207 (1997)).

Then, simian chymase used was obtained from the heart of rhesus monkey through purification in accordance with a Urata's human heart chymase purification method (which was reported in a document).

The inhibitory activity of the compounds of the present invention for chymase in vitro was obtained by the following methods.

Chymase activity was determined with reference to the method known in a literature (Miyazaki et al., Kekkan, Vol. 20, p. 207 (1997)). That is, the activity was measured by reacting free His-Leu formed together with Ang II with o-phthalaldehyde (hereinafter, abbreviated as OPT) to prepare a fluorescent derivative and determining the amount quantitatively by means of a fluorophotometer.

First, 3.6 µmol of each test compound was weighed in a test tube and was dissolved into 3 mL of DMSO. The DMSO solution was diluted 1000-fold with 20 mM Tris-hydrochloric acid buffer solution (pH 8.0) containing 0.01% Triton X-100 and 0.5 M potassium chloride to prepare 3.6×10⁻⁶ M solution, which was successively diluted with the buffer solution to prepare test sample solutions having concentrations of 3.6×10⁻⁶ M to 3.6×10⁻⁹ M. To 500 µL of the test sample solution of each concentration or buffer solution was added 50 µL of an enzyme solution, followed by 10 minutes of pre-incubation at 37°C. Then, 50 µL of 0.1 mM Ang I solution was added to initiate a reaction. Human angiotensin I (manufactured by SIGMA) was employed as Ang I. The enzyme (chymase) solution to be used for the reaction was adjusted so as to hydrolyze about 60% of substrate under the conditions, and the reaction wherein a buffer solution containing no enzyme was carried out as a blind test. After 120 minutes of incubation at 37°C, the reaction was terminated by adding 900 µL of trichloroacetic acid. Thereafter, the reaction mixture was centrifuged at 4°C at 3,000 rpm for 10 minutes and 2 mL of 2 N sodium hydroxide and 1 mL of methanol were added to 1 mL of the resulting supernatant. Thereto was added 100 µL of methanol solution containing 1.2 mg of N-acetyl-L-cysteine and 1 mg of OPT per 1 mL, whereby a derivatization reaction was initiated. After the reaction mixture was left on standing for exactly 1 hour, fluorescence intensity at fluorescence wavelength of 502 nm under excitation wavelength of 304 nm was measured. The measurement was repeated twice for each sample and blind test. The fluorescence intensity obtained by subtracting the average value at blind test from the average value thereof was determined as chymase activity.

In this regard, an enzymatic reaction using a buffer solution instead of the test sample solution was carried out as a control, and inhibitory ratio of chymase activity was determined as percentage by dividing the difference of subtraction of the activity at the addition of the test compound from the chymase activity at the control by the chymase activity at the control. Based on each inhibitory ratio, the concentration at which 50% of the activity was inhibited (hereinafter, referred to as IC₅₀ value) was calculated.

### Test Example 2

### Measurement of cathepsin G inhibitory activity and chymotrypsin inhibitory activity

Each activity of cathepsin G or chymotrypsin was measured by determining the amount of free p-nitroanilide quantitatively using a synthetic substrate which is colorless and yields colored products upon hydrolysis by means of a spectrophotometer. Chymotrypsin Type I-S derived from bovine pancreas was purchased from SIGMA. As cathepsin G, the product of Elastin Products Company, Inc. derived from human purulent sputum was used. Suc-Ala-Ala-Pro-Phe-pNA (manufactured by SIGMA) was used as the synthetic substrate. An inhibitory effect of a compound on each enzyme was determined by the following method.

Each test compound (5 µmol) was weighed in a test tube and dissolved into 2 mL of DMSO. The DMSO solution was diluted 100-fold with 20 mM Tris-hydrochloric acid buffer solution (pH 7.5) containing 0.01% Triton X-100 and 0.5 M potassium chloride to prepare 3.6×10⁻⁶ M solution, which was successively diluted to prepare test sample solutions having concentrations of 3.6×10⁻⁶ M to 3.6×10⁻⁹ M. To 200 µL of each test sample solution or buffer solution was added 100 µL of an enzyme solution of 40 µg/mL chymotrypsin or 8 units/mL cathepsin G, followed by 10 minutes of pre-incubation at 37°C. Then, 200 µL of 1 mM substrate solution was added to initiate an enzymatic reaction under a temperature of 37°C. A reaction wherein a buffer solution containing no enzyme was carried out as a blind test, and incubation time was 30 minutes and 60 minutes for chymotrypsin and cathepsin G, respectively. After the incubation, the reaction was terminated by adding 300 µL of 50% acetic acid, and absorbance at 405 nm was measured. The measurement was repeated twice for each sample and blind test. The absorbance obtained by subtracting the average value at blind test from the average value of each sample was determined as activity of each enzyme.

In this regard, an enzymatic reaction using a buffer solution instead of the test sample solution was carried out as a control, and inhibitory ratio of each enzyme activity was determined as percentage by dividing the difference from the subtraction of the activity at the addition of the test compound from the enzyme activity at the control by the enzyme activity at the control. Based on each inhibitory ratio, IC₅₀ value was calculated.

Table 3 shows IC₅₀ values of simian chymase inhibitory activity of the representative compounds as well as cathepsin G inhibitory activity and chymotrypsin inhibitory activity thereof.

**Table 4**

| Inhibitory specificity of active compounds | | | |
|---|---|---|---|
| Compound | IC₅₀ value (nmol/L) | | |
| | Chymase | Chymotrypsin | Cathepsin G |
| Chymostatin | 287 | 9.67 | 5.99 |
| Example 1 | 50 | >10000 | >10000 |
| Example 2 | 42 | | |
| Example 3 | 178 | | |
| Example 4 | 111 | >10000 | >10000 |
| Example 5 | 150 | >1000 | >1000 |
| Example 7 | 185 | >1000 | >1000 |
| Example 8 | 159 | >10000 | >10000 |
| Example 11 | 100 | | |
| Example 13 | 271 | | |
| Example 14 | 278 | >10000 | >10000 |
| Example 17 | 9 | >10000 | >10000 |
| Example 19 | 20 | | |
| Example 20 | 39 | | |
| Example 24 | 2 | >10000 | >10000 |
| Example 25 | 75 | | |
| Example 26 | 10 | >10000 | >10000 |
| Example 52 | 157 | | |

### Test Example 3

### Stability in rat plasma

Stability of the compounds of the invention in rat plasma was investigated.

Male SD rats (7-week-old) were anesthetized with ether under over-night fasting conditions and, after an abdominal part was incised, blood was collected from aorta abdominalis using a heparinized disposable plastic syringe. The blood was centrifuged under cooling to collect a supernatant plasma. The collected plasma was stored under freezing at -30°C and was melted before use. After a test compound was dissolved in DMSO, the solution was added to 200 µL of the plasma so as to be 10 µg/mL, followed by incubation at 37°C. After 60 minutes, the mixture was acidified by adding 200mL of 0.1 mol/L hydrochloric acid, and then extracted twice with 2 mL of ethyl acetate. The resulting organic layer was evaporated to dryness under a nitrogen gas flow and the residue was dissolved into 200 µL of acetonitrile to prepare a sample solution. On the other hand, the test compound was dissolved into 1% DMSO-acetonitrile so as to be 10 µg/mL as a control solution. The sample solution and control solution was investigated by high performance liquid chromatography (hereinafter, abbreviated as HPLC). Residual ratio (%) was determined as percentage by dividing the peak area of the sample solution by that of the control solution.

HPLC conditions:
Column: Waters Nova Pack C₁₈ (inner diameter: 3.9 mm, length: 150 mm)
Mobile phase A: acetonitrile/water = 10/90
Mobile phase B: acetonitrile/methanol = 50/50
Eluent: Mobile phase A-mobile phase B (100/0 to 0/100, linear gradient, 50 minutes)
Flow rate: 1.0 mL/minute
Amount of injection: 50 µL

Table 4 shows residual ratios of representative compounds in rat plasma.

**Table 5**

| Compound | Residual ratio in rat plasma (%) |
|---|---|
| Example 1 | 93.9 |
| Example 4 | 93.5 |
| Example 5 | 85.2 |
| Example 7 | 96.4 |
| Example 8 | 92.0 |
| Example 14 | 79.2 |
| Example 19 | 96.2 |
| Example 24 | 100 |
| Example 26 | 100 |

### INDUSTRIAL APPLICABILITY

The N-substituted benzothiophenesulfonamide derivatives or pharmaceutically acceptable salts thereof of the invention have a selective inhibitory action on chymase and are useful as agents for preventing or treating cardiac and circulatory diseases, especially cardiac infarction, restenosis after PTCA and intimal thickening after bypass grafting caused by abnormal increase of production of angiotensin II or endothelin I based on chymase activity.

## Claims

1. An N-substituted benzothiophenesulfonamide derivative represented by formula (I): 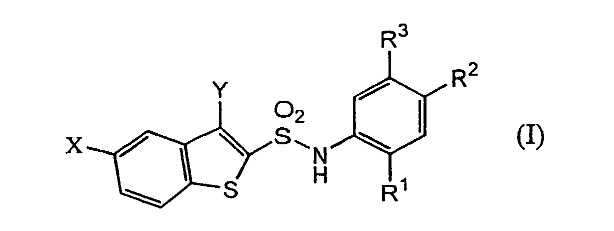 wherein X represents a hydrogen atom, a halogen atom or a lower alkyl group having 1 to 4 carbon atoms;
Y represents a lower alkyl group having 1 to 4 carbon atoms; R¹ and R² each may be the same or different and represents a hydrogen atom, a lower alkoxycarbonyl group having 1 to 4 carbon atoms in the alkoxy residue, a lower alkylsulfonyl group having 1 to 4 carbon atoms, a benzoyl group, an acyl group having 1 to 4 carbon atoms, a lower alkoxy group having 1 to 4 carbon atoms, a lower alkoxycarbonylmethylthioacetyl group having 1 to 4 carbon atoms in the alkoxy residue, a nitro group, -CONHR⁴ in which R⁴ represents a hydrogen atom, a lower alkoxycarbonylmethyl group having 1 to 4 carbon atoms in the alkoxy residue, a carboxymethyl group or -CH(CH₂OH)COOR⁵ in which R⁵ represents a hydrogen atom or a lower alkyl group having 1 to 4 carbon atoms, a group represented by formula: 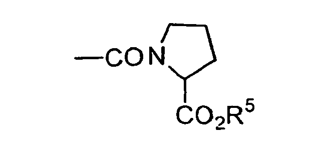 in which R⁵ has the same meaning as above, a monocyclic heterocyclic group represented by formulae which may be substituted by -CO₂R⁵ in which R⁵ has the same meaning as above: 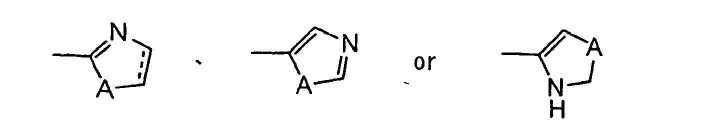 in which A represents an oxygen atom, a sulfur atom or NH and the dotted part represents a single bond or a double bond, a hydroxy lower alkyl group having 1 to 4 carbon atoms, a cyano group provided that R¹ and R² are not hydrogen atoms at the same time; and R³ represents a hydrogen atom, a lower alkoxy group having 1 to 4 carbon atoms or a lower alkyl group having 1 to 4 carbon atoms, except the compounds represented by formulae: 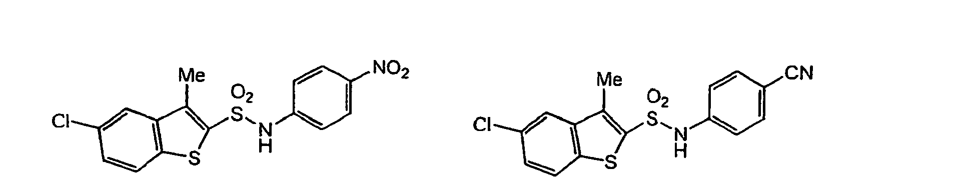 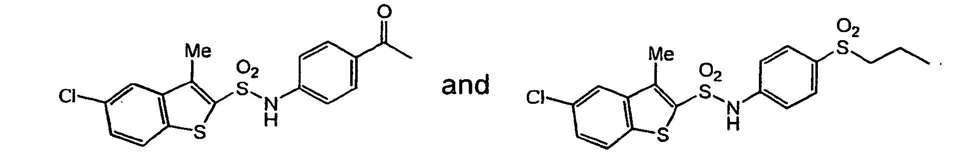 or a salt thereof.

2. The N-substituted benzothiophenesulfonamide derivative according to claim 1, wherein said derivative or a salt thereof is selected from the group consisting of methyl 4-(5-chloro-3-methylbenzo[b]thiophene-2-sulfonylamino)-3-methanesulfonylbenzoate, sodium methyl 4-(5-chloro-3-methylbenzo[b]thiophene-2-sulfonylamino)-3-methanesulfonylbenzoate, isopropyl 4-(5-chloro-3-methylbenzo[b]thiophene-2-sulfonylamino)-3-methanesulfonylbenzoate, 5-chloro-3-methylbenzo [b] thiophene-2-sulfonic acid (4-acetyl-2-methanesulfonylphenyl)amide, 5-chloro-3-methylbenzo[b]thiophene-2-sulfonic acid (4-benzoyl-2-methanesulfonylphenyl)amide, methyl 4-(5-fluoro-3-methylbenzo[b]thiophene-2-sulfonylamino)-3-methanesulfonylbenzoate, methyl 4-(5-methyl-3-methylbenzo[b]thiophene-2-sulfonylamino)-3-methanesulfonylbenzoate, 5-fluoro-3-methylbenzo[b]thiophene-2-sulfonic acid (4-acetyl-2-methanesulfonylphenyl)amide, methyl 4-(3-methylbenzo[b]thiophene-2-sulfonylamino)-3-methanesulfonylbenzoate, 2-[4-(5-chloro-3-methylbenzo[b]thiophene-2-sulfonylamino)-3-methanesulfonylphenyl]oxazole-4-carboxylic acid, 2-[4-(5-fluoro-3-methylbenzo[b]thiophene-2-sulfonylamino)-3-methanesulfonylphenyl]oxazole-4-carboxylic acid, disodium 2-[4-(5-chloro-3-methylbenzo[b]thiophene-2-sulfonylamino)-3-methanesulfonylphenyl]oxazole-4-carboxylate, and disodium 2-[4-(5-fluoro-3-methylbenzo[b]thiophene-2-sulfonylamino)-3-methanesulfonylphenyl]oxazole-4-carboxylate.

3. A pharmaceutical composition comprising N-substituted benzothiophenesulfonamide derivative represented by formula (I) : 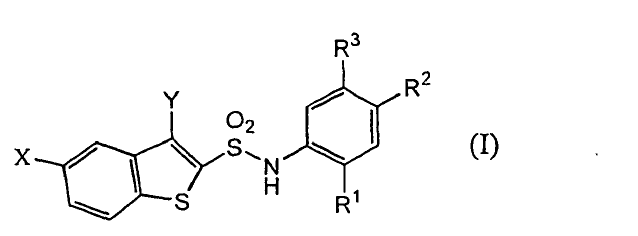 wherein X represents a hydrogen atom, a halogen atom or a lower alkyl group having 1 to 4 carbon atoms;
Y represents a lower alkyl group having 1 to 4 carbon atoms; R¹ and R² each may be the same or different and represents a hydrogen atom, a lower alkoxycarbonyl group having 1 to 4 carbon atoms in the alkoxy residue, a lower alkylsulfonyl group having 1 to 4 carbon atoms, a benzoyl group, an acyl group having 1 to 4 carbon atoms, a lower alkoxy group having 1 to 4 carbon atoms, a lower alkoxycarbonylmethylthioacetyl group having 1 to 4 carbon atoms in the alkoxy residue, a nitro group, -CONHR⁴ in which R⁴ represents a hydrogen atom, a lower alkoxycarbonylmethyl group having 1 to 4 carbon atoms in the alkoxy residue, a carboxymethyl group or -CH(CH₂OH)COOR⁵ in which R⁵ represents a hydrogen atom or a lower alkyl group having 1 to 4 carbon atoms, a group represented by formula: 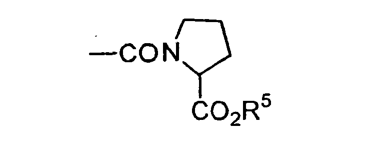 in which R⁵ has the same meaning as above, a monocyclic heterocyclic group represented by formulae which may be substituted by -CO₂R⁵ in which R⁵ has the same meaning as above: 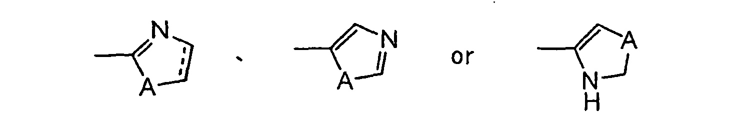 in which A represents an oxygen atom, a sulfur atom or NH and the dotted part represents a single bond or a double bond, a hydroxy lower alkyl group having 1 to 4 carbon atoms, a cyano group provided that R¹ and R² are not hydrogen atoms at the same time; and R³ represents a hydrogen atom, a lower alkoxy group having 1 to 4 carbon atoms or a lower alkyl group having 1 to 4 carbon atoms, or a salt thereof.

4. An agent for preventing or treating cardiac infarction, restenosis after percutaneous transluminal coronary angioplasty, and intimal thickening after bypass grafting, wherein the agent comprises the N-substituted benzothiophenesulfonamide derivative or a salt thereof as defined in claim 3.

5. A N-substituted benzothiophenesulfonamide derivative represented by formula (I): 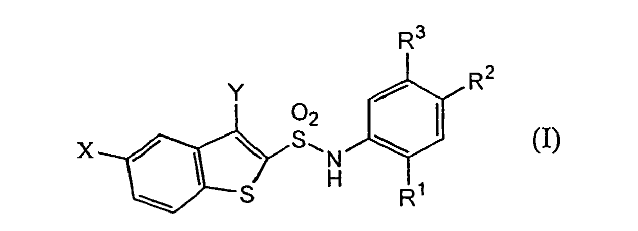 wherein X represents a hydrogen atom, a halogen atom or a lower alkyl group having 1 to 4 carbon atoms;
Y represents a lower alkyl group having 1 to 4 carbon atoms; R¹ and R² each may be the same or different and represents a hydrogen atom, a lower alkoxycarbonyl group having 1 to 4 carbon atoms in the alkoxy residue, a lower alkylsulfonyl group having 1 to 4 carbon atoms, a benzoyl group, an acyl group having 1 to 4 carbon atoms, a lower alkoxy group having 1 to 4 carbon atoms, a lower alkoxycarbonylmethylthioacetyl group having 1 to 4 carbon atoms in the alkoxy residue, a nitro group, -CONHR⁴ in which R⁴ represents a hydrogen atom, a lower alkoxycarbonylmethyl group having 1 to 4 carbon atoms in the alkoxy residue, a carboxymethyl group or -CH(CH₂OH)COOR⁵ in which R⁵ represents a hydrogen atom or a lower alkyl group having 1 to 4 carbon atoms, a group represented by formula: 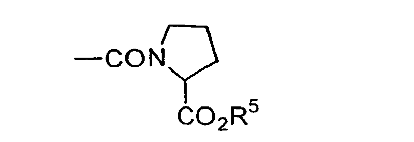 in which R⁵ has the same meaning as above, a monocyclic heterocyclic group represented by formulae which may be substituted by -CO₂R⁵ in which R⁵ has the same meaning as above: 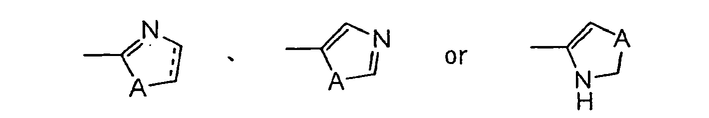 in which A represents an oxygen atom, a sulfur atom or NH and the dotted part represents a single bond or a double bond, a hydroxy lower alkyl group having 1 to 4 carbon atoms, a cyano group provided that R¹ and R² are not hydrogen atoms at the same time; and R³ represents a hydrogen atom, a lower alkoxy group having 1 to 4 carbon atoms or a lower alkyl group having 1 to 4 carbon atoms, or a salt thereof for preventing or treating hypertension, hypercardia, cardiac failure, cardiac infarction, arteriosclerosis, diabetic or non-diabetic renal diseases, diabetic retinopathy, restenosis after percutaneous transluminal coronary angioplasty, intimal thickening after bypass grafting, ischemic re-perfusion disorder, chronic rheumatism, keloid, psoriasis, allergy, inflammation, asthma, atopic dermatitis and solid tumors caused by abnormal increase of production of angiotensin II or endothelin I.

## Patentansprüche

1. N-Substituiertes Benzothiophensulfonamidderivat, dargestellt durch die Formel (I): 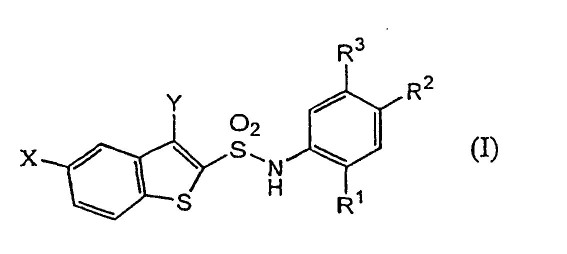 wobei X ein Wasserstoffatom, ein Halogenatom oder eine Niedrigalkylgruppe mit 1 bis 4 Kohlenstoffatomen darstellt;
Y stellt eine Niedrigalkylgruppe mit 1 bis 4 Kohlenstoffatomen dar;
R¹ und R² können jeweils gleich oder verschieden sein und stellen ein Wasserstoffatom dar, eine Niedrigalkoxycarbonylgruppe mit 1 bis 4 Kohlenstoffatomen im Alkoxyrest, eine Niedrigalkylsulfonylgruppe mit 1 bis 4 Kohlenstoffatomen, eine Benzoylgruppe, eine Acylgruppe mit 1 bis 4 Kohlenstoffatomen, eine Niedrigalkoxygruppe mit 1 bis 4 Kohlenstoffatomen, eine Niedrigalkoxycarbonylmethylthioacetylgruppe mit 1 bis 4 Kohlenstoffatomen im Alkoxyrest, eine Nitrogruppe, -CONHR⁴, wobei R⁴ ein Wasserstoffatom darstellt, eine Niedrigalkoxycarbonylmethylgruppe mit 1 bis 4 Kohlenstoffatomen im Alkoxyrest, eine Carboxylmethylgruppe oder -CH(CH₂OH)COOR⁵, wobei R⁵ ein Wasserstoffatom darstellt, oder eine Niedrigalkylgruppe mit 1 bis 4 Kohlenstoffatomen, eine Gruppe, dargestellt durch die Formel: 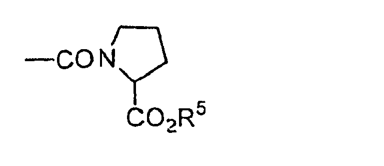 wobei R⁵ dieselbe Bedeutung wie oben hat, eine monocyclische heterocyclische Gruppe, dargestellt durch Formeln, die durch -CO₂R⁵ substituiert sein können, wobei R⁵ dieselbe Bedeutung wie oben hat: 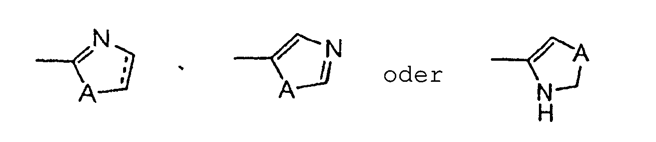 wobei A ein Sauerstoffatom darstellt, ein Schwefelatom oder NH, und der gestrichelte Teil stellt eine Einfachbindung oder eine Doppelbindung dar, eine Hydroxyniedrigalkylgruppe mit 1 bis 4 Kohlenstoffatomen, eine Cyanogruppe, vorausgesetzt, dass R¹ und R² nicht gleichzeitig Wasserstoffatome sind; und R³ stellt ein Wasserstoffatom dar, eine Niedrigalkoxygruppe mit 1 bis 4 Kohlenstoffatomen oder eine Niedrigalkylgruppe mit 1 bis 4 Kohlenstoffatomen, mit Ausnahme der Verbindungen, dargestellt durch die Formeln: 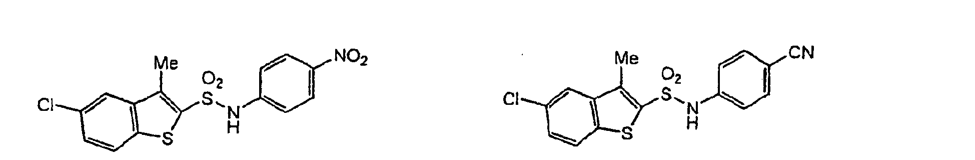 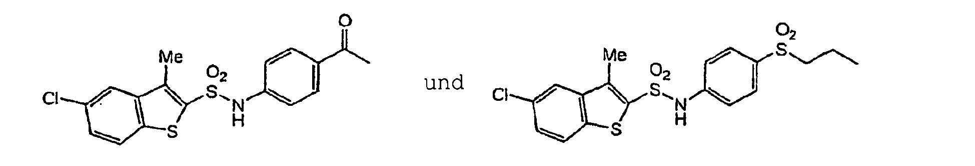 oder ein Salz davon.

2. N-Substituiertes Benzothiophensulfonamidderivat gemäß Anspruch 1, wobei das Derivat oder Salz davon ausgewählt ist aus der Gruppe, bestehend aus Methyl-4-(5-chlor-3-methylbenzo[b]thiophen-2-sulfonylamino)-3-methansulfonylbenzoat, Natriummethyl-4-(5-chlor-3-methylbenzo[b]thiophen-2-sulfonylamino)-3-methansulfonylbenzoat, Isopropyl-4-(5-chlor-3-methylbenzo[b]thiophen-2-sulfonylamino)-3-methansulfonylbenzoat, 5-Chlor-3-methylbenzo[b]thiophen-2-sulfonsäure-(4-acetyl-2-methansulfonylphenyl)amid, 5-Chlor-3-methylbenzo[b]-thiophen-2-sulfonsäure-(4-benzoyl-2-methansulfonylphenyl)-amid, Methyl-4-(5-fluor-3-methylbenzo[b]thiophen-2-sulfonylamino)-3-methansulfonylbenzoat, Methyl-4-(5-methyl-3-methylbenzo[b]thiophen-2-sulfonylamino)-3-methansulfonylbenzoat, 5-Fluor-3-methylbenzo[b]thiophen-2-sulfonsäure-(4-acetyl-2-methansulfonylphenyl)amid, Methyl-4-(3-methylbenzo[b]thiophen-2-sulfonylamino)-3-methansulfonylbenzoat, 2-[4-(5-Chlor-3-methylbenzo[b]thiophen-2-sulfonylamino)-3-methansulfonylphenyl]oxazol-4-carbonsäure, 2-[4-(5-Fluor-3-methylbenzo[b]-thiophen-2-sulfonylamino)-3-methansulfonylphenyl]oxazol-4-carbonsäure, Dinatrium-2-[4-(5-chlor-3-methylbenzo[b]thiophen-2-sulfonylamino)-3-methansulfonylphenyl]oxazol-4-carboxylat und Dinatrium-2-[4-(5-fluor-3-methylbenzo[b]thiophen-2-sulfonylamino)-3-methansulfonylphenyl]oxazol-4-carboxylat.

3. Pharmazeutische Zusammensetzung, umfassend ein N-substituiertes Benzothiophensulfonamidderivat, dargestellt durch die Formel (I): 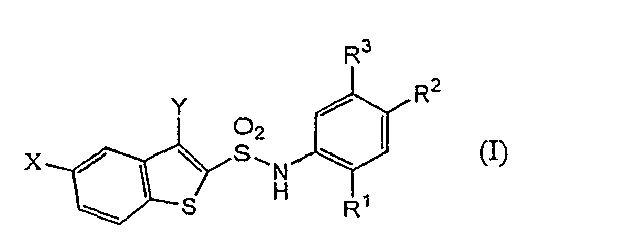 wobei X ein Wasserstoffatom, ein Halogenatom oder eine Niedrigalkylgruppe mit 1 bis 4 Kohlenstoffatomen darstellt;
Y stellt eine Niedrigalkylgruppe mit 1 bis 4 Kohlenstoffatomen dar;
R¹ und R² können jeweils gleich oder verschieden sein und stellen ein Wasserstoffatom dar, eine Niedrigalkoxycarbonylgruppe mit 1 bis 4 Kohlenstoffatomen im Alkoxyrest, eine Niedrigalkylsulfonylgruppe mit 1 bis 4 Kohlenstoffatomen, eine Benzoylgruppe, eine Acylgruppe mit 1 bis 4 Kohlenstoffatomen, eine Niedrigalkoxygruppe mit 1 bis 4 Kohlenstoffatomen, eine Niedrigalkoxycarbonylmethylthioacetylgruppe mit 1 bis 4 Kohlenstoffatomen im Alkoxyrest, eine Nitrogruppe, -CONHR⁴, wobei R⁴ ein Wasserstoffatom darstellt, eine Niedrigalkoxycarbonylmethylgruppe mit 1 bis 4 Kohlenstoffatomen im Alkoxyrest, eine Carboxylmethylgruppe oder -CH(CH₂OH)COOR⁵, wobei R⁵ ein Wasserstoffatom darstellt, oder eine Niedrigalkylgruppe mit 1 bis 4 Kohlenstoffatomen, eine Gruppe, dargestellt durch die Formel: 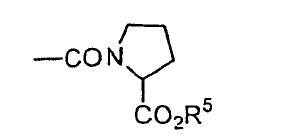 wobei R⁵ dieselbe Bedeutung wie oben hat, eine monocyclische heterocyclische Gruppe, dargestellt durch Formeln, die durch -CO₂R⁵ substituiert sein können, wobei R⁵ dieselbe Bedeutung wie oben hat: 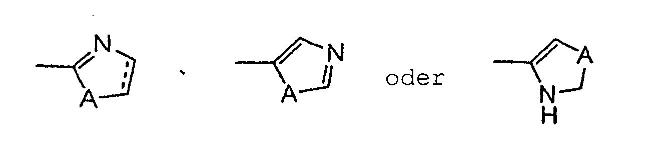 wobei A ein Sauerstoffatom darstellt, ein Schwefelatom oder NH, und der gestrichelte Teil stellt eine Einfachbindung oder eine Doppelbindung dar, eine Hydroxyniedrigalkylgruppe mit 1 bis 4 Kohlenstoffatomen, eine Cyanogruppe, vorausgesetzt, dass R¹ und R² nicht gleichzeitig Wasserstoffatome sind; und R³ stellt ein Wasserstoffatom dar, eine Niedrigalkoxygruppe mit 1 bis 4 Kohlenstoffatomen oder eine Niedrigalkylgruppe mit 1 bis 4 Kohlenstoffatomen, oder ein Salz davon.

4. Mittel zur Prävention oder Behandlung von Herzinfarkt, Restinose nach perkutaner transluminaler koronarer Angioplastie und Intimaverdickung nach Bypassoperation, wobei das Mittel das N-substituierte Benzothiophensulfonamidderivat oder ein Salz davon, wie in Anspruch 3 definiert, umfasst.

5. N-substituiertes Benzothiophensulfonamidderivat, dargestellt durch Formel (I): 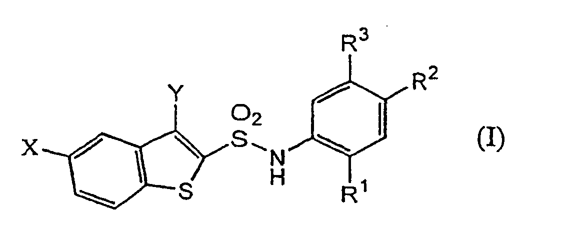 wobei X ein Wasserstoffatom, ein Halogenatom oder eine Niedrigalkylgruppe mit 1 bis 4 Kohlenstoffatomen darstellt;
Y stellt eine Niedrigalkylgruppe mit 1 bis 4 Kohlenstoffatomen dar;
R¹ und R² können jeweils gleich oder verschieden sein und stellen ein Wasserstoffatom dar, eine Niedrigalkoxycarbonylgruppe mit 1 bis 4 Kohlenstoffatomen im Alkoxyrest, eine Niedrigalkylsulfonylgruppe mit 1 bis 4 Kohlenstoffatomen, eine Benzoylgruppe, eine Acylgruppe mit 1 bis 4 Kohlenstoffatomen, eine Niedrigalkoxygruppe mit 1 bis 4 Kohlenstoffatomen, eine Niedrigalkoxycarbonylmethylthioacetylgruppe mit 1 bis 4 Kohlenstoffatomen im Alkoxyrest, eine Nitrogruppe, -CONHR⁴, wobei R⁴ ein Wasserstoffatom darstellt, eine Niedrigalkoxycarbonylmethylgruppe mit 1 bis 4 Kohlenstoffatomen im Alkoxyrest, eine Carboxylmethylgruppe oder -CH(CH₂OH)COOR⁵, wobei R⁵ ein Wasserstoffatom darstellt, oder eine Niedrigalkylgruppe mit 1 bis 4 Kohlenstoffatomen, eine Gruppe, dargestellt durch die Formel: 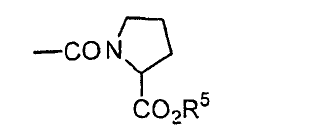 wobei R⁵ dieselbe Bedeutung wie oben hat, eine monocyclische heterocyclische Gruppe, dargestellt durch Formeln, die durch -CO₂R⁵ substituiert sein können, wobei R⁵ dieselbe Bedeutung wie oben hat: 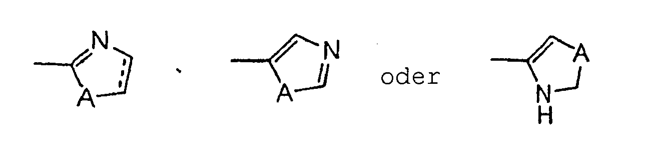 wobei A ein Sauerstoffatom darstellt, ein Schwefelatom oder NH, und der gestrichelte Teil stellt eine Einfachbindung oder eine Doppelbindung dar, eine Hydroxyniedrigalkylgruppe mit 1 bis 4 Kohlenstoffatomen, eine Cyanogruppe, vorausgesetzt, dass R¹ und R² nicht gleichzeitig Wasserstoffatome sind; und R³ stellt ein Wasserstoffatom dar, eine Niedrigalkoxygruppe mit 1 bis 4 Kohlenstoffatomen oder eine Niedrigalkylgruppe mit 1 bis 4 Kohlenstoffatomen, oder ein Salz davon, zur Prävention oder Behandlung von Bluthochdruck, Herzhypertrophie, Herzschwäche, Herzinfarkt, Arteriosklerose, diabetischen oder nichtdiabetischen Nierenkrankheiten, diabetischer Retinopathie, Restenose nach perkutaner transluminaler koronarer Angioplastie, Intimaverdickung nach Bypassoperation, ischämischer Reperfusionsstörung, chronischem Rheuma, Keloid, Psoriasis, Allergie, Entzündung, Asthma, atopischer Dermatitis und soliden Tumoren, verursacht durch abnormale Zunahme der Produktion von Angiotensin II oder Endothelin I.

## Revendications

1. Dérivé de benzothiophènesulfonamide N-substitué représenté par la formule (I) : 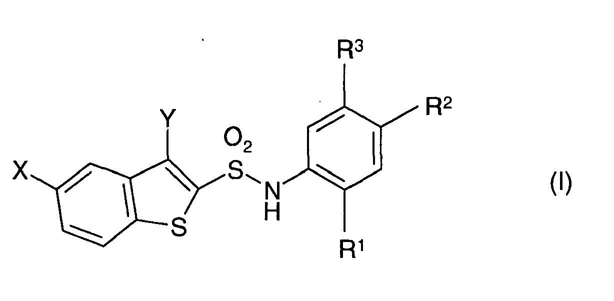 où X représente un atome d'hydrogène, un atome d'halogène ou un groupe alkyle inférieur ayant 1 à 4 atomes de carbone ;
Y représente un groupe alkyle inférieur ayant 1 à 4 atomes de carbone ;
R¹ et R² peuvent être chacun identiques ou différents et représentent un atome d'hydrogène, un groupe alcoxycarbonyle inférieur ayant 1 à 4 atomes de carbone dans le groupement alcoxy, un groupe alkylsulfonyle inférieur ayant 1 à 4 atomes de carbone, un groupe benzoyle, un groupe acyle ayant 1 à 4 atomes de carbone, un groupe alcoxy inférieur ayant 1 à 4 atomes de carbone, un groupe alcoxycarbonylméthylthioacétyle inférieur ayant 1 à 4 atomes de carbone dans le groupement alcoxy, un groupe nitro, -CONHR⁴ où R⁴ représente un atome d'hydrogène, un groupe alcoxycarbonylméthyle inférieur ayant 1 à 4 atomes de carbone dans le groupement alcoxy, un groupe carboxyméthyle ou -CH(CH₂OH)COOR⁵ où R⁵ représente un atome d'hydrogène ou un groupe alkyle inférieur ayant 1 à 4 atomes de carbone, un groupe représenté par la formule : 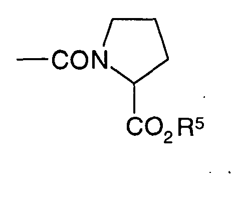 où R⁵ a la même signification que ci-dessus, un groupe hétérocyclique monocyclique représenté par les formules qui peuvent être substituées par -CO₂R⁵ où R⁵ a la même signification que ci-dessus : 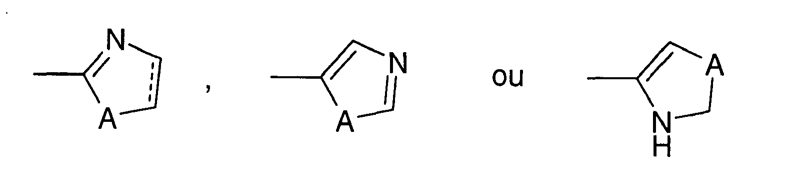 où A représente un atome d'oxygène, un atome de soufre ou NH et la partie pointillée représente une simple liaison ou une double liaison, un groupe hydroxyalkyle inférieur ayant 1 à 4 atomes de carbone, un groupe cyano à condition que R¹ et R² ne soient pas des atomes d'hydrogène en même temps ; et R³ représente un atome d'hydrogène, un groupe alcoxy inférieur ayant 1 à 4 atomes de carbone ou un groupe alkyle inférieur ayant 1 à 4 atomes de carbone, à l'exception des composés représentés par les formules : 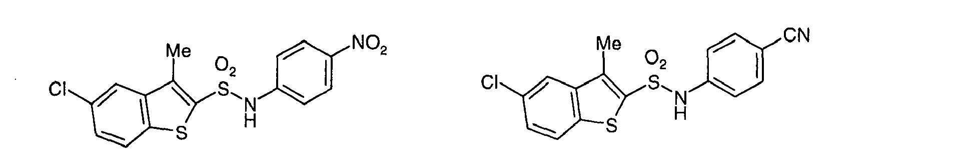 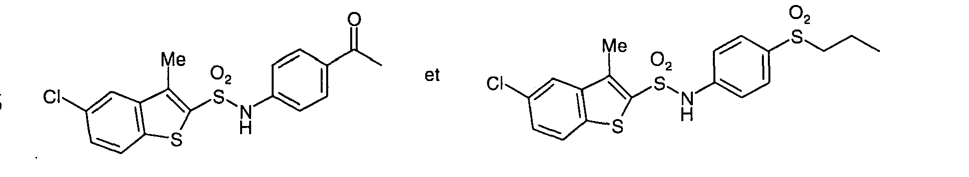 ou sel de celui-ci.

2. Dérivé de benzothiophènesulfonamide N-substitué selon la revendication 1 où ledit dérivé ou un sel de celui-ci est choisi dans le groupe consistant en 4-(5-chloro-3-méthylbenzo[b]thiophène-2-sulfonylamino)-3-méthanesulfonylbenzoate de méthyle, 4-(5-chloro-3-méthylbenzo[b]thiophène-2-sulfonylamino)-3-méthanesulfonylbenzoate de méthyle sodique, 4-(5-chloro-3-méthylbenzo[b]thiophène-2-sulfonylamino)-3-méthanesulfonylbenzoate d'isopropyle, (4-acétyl-2-méthanesulfonylphényl)amide d'acide 5-chloro-3-méthylbenzo[b]-thiophène-2-sulfonique, (4-benzoyl-2-méthanesulfonylphényl)amide d'acide 5-chloro-3-méthylbenzo[b]thiophène-2-sulfonique, 4-(5-fluoro-3-méthylbenzo[b]thiophène-2-sulfonylamino)-3-méthanesulfonylbenzoate de méthyle, 4-(5-méthyl-3-méthylbenzo[b]thiophène-2-sulfonylamino)-3-méthanesulfonylbenzoate de méthyle, (4-acétyl-2-méthanesulfonylphényl)amide d'acide 5-fluoro-3-méthylbenzo[b]-thiophène2-sulfonique, 4-(3-méthylbenzo[b]thiophène-2-sulfonylamino)-3-méthanesulfonylbenzoate de méthyle, acide 2-[4-(5-chloro-3-méthylbenzo[b]thiophène-2-sulfonylamino)-3-méthanesulfonylphényl]-oxazole-4-carboxylique, acide 2-[4-(5-fluoro-3-méthylbenzo[b]-thiophène-2-sulfonylamino)-3-méthanesulfonylphényl]-oxazole-4-carboxylique, 2-[4-(5-chloro-3-méthylbenzo[b]thiophène-2-sulfonylamino)-3-méthanesulfonylphényl]oxazole-4-carboxylate de disodium, et 2-[4-(5-fluoro-3-méthylbenzo[b]thiophène-2-sulfonyl-amino)-3-méthanesulfonylphényl]oxazole-4-carboxylate de disodium.

3. Composition pharmaceutique comprenant un dérivé de benzothiophènesulfonamide N-substitué représenté par la formule (I) : 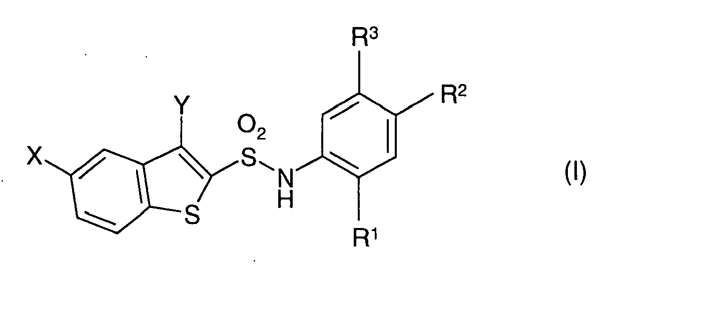 où X représente un atome d'hydrogène, un atome d'halogène ou un groupe alkyle inférieur ayant 1 à 4 atomes de carbone ;
Y représente un groupe alkyle inférieur ayant 1 à 4 atomes de carbone ;
R¹ et R² peuvent être chacun identiques ou différents et représentent un atome d'hydrogène, un groupe alcoxycarbonyle inférieur ayant 1 à 4 atomes de carbone dans le groupement alcoxy, un groupe alkylsulfonyle inférieur ayant 1 à 4 atomes de carbone, un groupe benzoyle, un groupe acyle ayant 1 à 4 atomes de carbone, un groupe alcoxy inférieur ayant 1 à 4 atomes de carbone, un groupe alcoxycarbonylméthylthioacétyle inférieur ayant 1 à 4 atomes de carbone dans le groupement alcoxy, un groupe nitro, -CONHR⁴ où R⁴ représente un atome d'hydrogène, un groupe alcoxycarbonylméthyle inférieur ayant 1 à 4 atomes de carbone dans le groupement alcoxy, un groupe carboxyméthyle ou -CH(CH₂OH)COOR⁵ où R⁵ représente un atome d'hydrogène ou un groupe alkyle inférieur ayant 1 à 4 atomes de carbone, un groupe représenté par la formule : 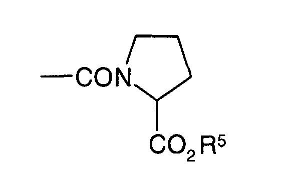 où R⁵ a la même signification que ci-dessus, un groupe hétérocyclique monocyclique représenté par les formules qui peuvent être substituées par -CO₂R⁵ où R⁵ a la même signification que ci-dessus : 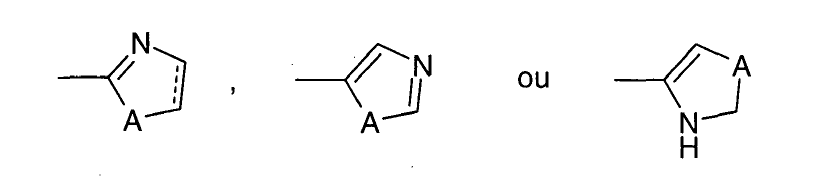 où A représente un atome d'oxygène, un atome de soufre ou NH et la partie pointillée représente une simple liaison ou une double liaison, un groupe hydroxyalkyle inférieur ayant 1 à 4 atomes de carbone, un groupe cyano à condition que R¹ et R² ne soient pas des atomes d'hydrogène en même temps ; et R³ représente un atome d'hydrogène, un groupe alcoxy inférieur ayant 1 à 4 atomes de carbone ou un groupe alkyle inférieur ayant 1 à 4 atomes de carbone, ou sel de celui-ci.

4. Agent pour prévenir ou traiter un infarctus du myocarde, une resténose après une angioplastie coronaire transluminale percutanée et un épaississement de l'intima après un pontage, où l'agent comprend le dérivé de benzothiophènesulfonamide N-substitué ou un sel de celui-ci selon la revendication 3.

5. Dérivé de benzothiophènesulfonamide N-substitué représenté par la formule (I) : 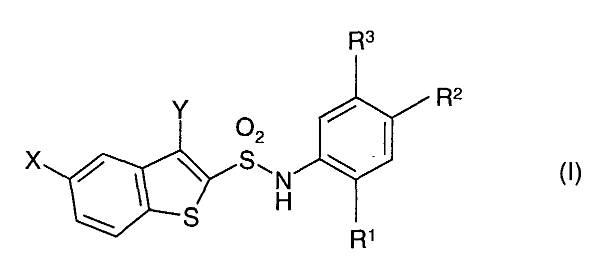 où X représente un atome d'hydrogène, un atome d'halogène ou un groupe alkyle inférieur ayant 1 à 4 atomes de carbone ;
Y représente un groupe alkyle inférieur ayant 1 à 4 atomes de carbone ;
R¹ et R² peuvent être chacun identiques ou différents et représentent un atome d'hydrogène, un groupe alcoxycarbonyle inférieur ayant 1 à 4 atomes de carbone dans le groupement alcoxy, un groupe alkylsulfonyle inférieur ayant 1 à 4 atomes de carbone, un groupe benzoyle, un groupe acyle ayant 1 à 4 atomes de carbone, un groupe alcoxy inférieur ayant 1 à 4 atomes de carbone, un groupe alcoxycarbonylméthylthioacétyle inférieur ayant 1 à 4 atomes de carbone dans le groupement alcoxy, un groupe nitro, -CONHR⁴ où R⁴ représente un atome d'hydrogène, un groupe alcoxycarbonylméthyle inférieur ayant 1 à 4 atomes de carbone dans le groupement alcoxy, un groupe carboxyméthyle ou -CH(CH₂OH)COOR⁵ où R⁵ représente un atome d'hydrogène ou un groupe alkyle inférieur ayant 1 à 4 atomes de carbone, un groupe représenté par la formule : 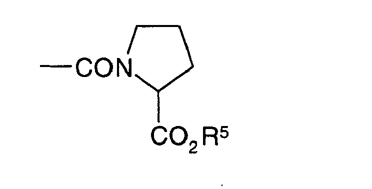 où R⁵ a la même signification que ci-dessus, un groupe hétérocyclique monocyclique représenté par les formules qui peuvent être substituées par -CO₂R⁵ où R⁵ a la même signification que ci-dessus : 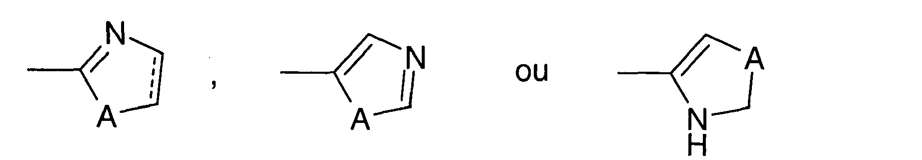 où A représente un atome d'oxygène, un atome de soufre ou NH et la partie pointillée représente une simple liaison ou une double liaison, un groupe hydroxyalkyle inférieur ayant 1 à 4 atomes de carbone, un groupe cyano à condition que R¹ et R² ne soient pas des atomes d'hydrogène en même temps ; et R³ représente un atome d'hydrogène, un groupe alcoxy inférieur ayant 1 à 4 atomes de carbone ou un groupe alkyle inférieur ayant 1 à 4 atomes de carbone, ou sel de celui-ci pour prévenir ou traiter l'hypertension, l'hypercardie, l'insuffisance cardiaque, l'infarctus du myocarde, l'artériosclérose, les maladies rénales diabétiques ou non diabétiques, la rétinopathie diabétique, la resténose après une angioplastie coronaire transluminale percutanée, un épaississement de l'intima après un pontage, un trouble de reperfusion ischémique, le rhumatisme chronique, la chéloïde, le psoriasis, l'allergie, l'inflammation, l'asthme, la dermatite atopique et les tumeurs solides causées par une augmentation anormale de la production d'angiotensine II ou d'endothéline I.
